# EUROPÄISCHE PATENTSCHRIFT

(11) **EP 4 070 843 B1**
(45) Veröffentlichungstag und Bekanntmachung des Hinweises auf die Patenterteilung: **16.07.2025**
(21) Anmeldenummer: 22163682.2
(22) Anmeldetag: 22.03.2022
(51) Int. Cl.: A61M 16/06, A61M 16/08

(54) **AUSATEMSYSTEM UND KUGELGELENK FÜR EINE PATIENTENSCHNITTSTELLE**
EXHALATION SYSTEM AND BALL JOINT FOR PATIENT INTERFACE
SYSTÈME D'EXHALATION ET JOINT À ROTULE POUR UNE INTERFACE PATIENT

(30) Priorität: 09.04.2021 DE 102021001834
(43) Veröffentlichungstag der Anmeldung: 12.10.2022
(73) Patentinhaber: Löwenstein Medical Technology S.A., 2557 Luxembourg (LU)
(72) Erfinder: Bechtel, Martin, 21423 Winsen / Luhe (DE); Gardein, Joachim, 38430 Icod de los Vinos (ES); Frerichs, Arnold, 21614 Buxtehude (DE)
(74) Vertreter: Löwenstein Medical IP

(56) Entgegenhaltungen:
- EP-A1- 3 305 354
- WO-A1-2014/129913
- WO-A1-2017/049357
- WO-A1-2020/135760
- DE-T5- 112015 003 876
- US-A- 5 921 239

## Beschreibung

Die Erfindung betrifft ein Ausatemsystem für eine Patientenschnittstelle sowie eine Patientenschnittstelle mit einem solchen Ausatemsystem und einem Kugelgelenk.

Im Zuge der Beatmung oder Atmungsunterstützung mit Beatmungsgeräten stellen Patientenschnittstellen das Bindeglied zwischen Patient/Anwender und Gerät dar. Bei Verwendung eines Ein-Schlauch-Systems wird ein Teil des ausgeatmeten Atemgases in den Schlauch beziehungsweise die Patientenschnittstelle geatmet. Um eine Akkumulation von CO2 in dem Atemgas zu vermeiden, wird das Atemgas häufig zumindest zum Teil direkt aus der Patientenschnittstelle in die Umgebung geleitet. Das Strömen des Atemgases in die Umgebung führt dabei zu Geräuschen, welche besonders für den Anwender/Patienten unangenehm und störend sein können. Auch für einen etwaigen Bettpartner kann das ausströmende Atemgas störend sein, etwa wenn die Luft den etwaigen Bettpartner trifft.

WO 2017/049357 A1 beschreibt eine Atemmaske mit einem Kugelgelenk und einem Scharnier. Die Atemmaske umfasst einen Kanal zum Auswaschen von Atemgas.

Weitere Beispiele für Atemmasken mit einem Kugelgelenk sind in WO 2020/135760 A1, DE 11 2015 003 876 T5, US5921239A und WO 2014/129913 A1 beschrieben.

EP 3 305 354 A1 beschreibt eine alternative Ausführungsform einer Atemmaske mit mehreren Strömungswegen.

Aufgabe der vorliegenden Erfindung ist daher die Bereitstellung einer leisen und komfortablen Patientenschnittstelle. Die Aufgabe wird durch das Ausatemsystem gemäß Anspruch 1 gelöst. Auch wird die Aufgabe durch die Patientenschnittstelle gemäß Anspruch 18 gelöst.

Das Ausatemsystem für eine Patientenschnittstelle umfasst zumindest zwei Wände, wobei die Wände zumindest abschnittsweise nebeneinander angeordnet sind. Das Ausatemsystem weist zumindest zwei Strömungswege zur Strömung von Atemgas aus einem Innenraum der Patientenschnittstelle auf, wobei der erste Strömungsweg zumindest teilweise zwischen der ersten Wand und der zweiten Wand verläuft und der erste Strömungsweg zum zumindest zeitweisen Abbau von Atemgasdruck ausgebildet ist und wobei der zweite Strömungsweg zumindest teilweise von der zweiten Wand umschlossen wird und der zweite Strömungsweg zumindest teilweise zur zumindest zeitweisen Strömung von Atemgas in den Innenraum der Patientenschnittstelle ausgebildet ist. In zumindest einer der Wände sind zumindest vier Rillenanordnungen angeordnet, wobei jede Rillenanordnung zumindest zwei Rillen umfasst und wobei die Rillen zusammen mit der anderen Wand Kanäle ausbilden, wobei die Rillenanordnungen immer paarweise angeordnet sind und der Abstand zwischen zwei Paaren von Rillenanordnungen größer ist als der Abstand der zwei Rillenanordnungen eines jeweiligen Paars untereinander.

In manchen Ausführungsformen kennzeichnet das Ausatemsystem, dass das Ausatemsystem eine dritte Wand umfasst, wobei die dritte Wand den zweiten Strömungsweg zumindest teilweise umschließt.

In manchen Ausführungsformen kennzeichnet das Ausatemsystem, dass der zweite Strömungsweg zumindest zeitweise einen Abzweig aufweist, wobei der Abzweig zwischen der der zweiten Wand und der dritten Wand zumindest abschnittsweise koaxial zu dem zweiten Strömungsweg verläuft und zur zumindest zeitweisen Strömung von Atemgas aus dem Innenraum der Patientenschnittstelle und zum zumindest zeitweisen Abbau von Atemgasdruck ausgebildet ist.

In manchen Ausführungsformen kennzeichnet das Ausatemsystem, dass der erste Strömungsweg zumindest teilweise koaxial zum zweiten Strömungsweg verläuft.

In manchen Ausführungsformen kennzeichnet das Ausatemsystem, dass der erste Strömungsweg, der zweite Strömungsweg und der Abzweig zumindest teilweise koaxial zueinander verlaufen, wobei der zweite Strömungsweg zentral verläuft und der Abzweig zumindest teilweise um den zweiten Strömungsweg verläuft und wobei der erste Strömungsweg zumindest teilweise um den zweiten Strömungsweg und den Abzweig verläuft.

In manchen Ausführungsformen kennzeichnet das Ausatemsystem, dass der erste Strömungsweg zumindest einen Teilströmungsweg aufweist, welcher im Wesentlichen parallel zum ersten Strömungsweg verläuft.

In manchen Ausführungsformen kennzeichnet das Ausatemsystem, dass die zumindest eine Rillenanordnung in einer Innenfläche der ersten Wand angeordnet ist und die Rillen zusammen mit einer Außenfläche der zweiten Wand die Kanäle bilden oder zumindest eine Rillenanordnung in der Außenfläche der zweiten Wand angeordnet ist und die Rillen zusammen mit Innenfläche der ersten Wand die Kanäle bilden.

In manchen Ausführungsformen kennzeichnet das Ausatemsystem, dass durch die Kanäle die Teilströmungswege des ersten Strömungsweges verlaufen.

In manchen Ausführungsformen kennzeichnet das Ausatemsystem, dass die Kanäle den Innenraum der Patientenschnittstelle mit dem Außenbereich der Patientenschnittstelle gasleitend verbinden.

In manchen Ausführungsformen kennzeichnet das Ausatemsystem, dass an der Außenfläche der zweiten Wand ein Kragen angeordnet ist, wobei der Kragen mit einer Unterseite formschlüssig auf einer oberen Fläche der ersten Wand angeordnet ist, und wobei die Unterseite des Kragens und/oder die obere Fläche der ersten Wand zumindest eine Aussparung aufweist, welche zusammen mit der oberen Fläche der ersten Wand und/oder der Unterseite des Kragens zumindest einen Spalt bildet.

In manchen Ausführungsformen kennzeichnet das Ausatemsystem, dass an der Unterseite des Kragens eine Verlängerung des ersten Strömungsweges verläuft und in den Spalten zumindest teilweise eine Verlängerung der Teilströmungswege verläuft.

In manchen Ausführungsformen kennzeichnet das Ausatemsystem, dass der Strömungsquerschnitt der Teilströmungswege nicht gleich dem Querschnitt der Verlängerung ist.

In manchen Ausführungsformen kennzeichnet das Ausatemsystem, dass sich die zumindest eine Aussparung an der Unterseite des Kragens von einer Außenfläche des Kragens bis zur Außenfläche der zweiten Wand erstreckt.

In manchen Ausführungsformen kennzeichnet das Ausatemsystem, dass an der Außenfläche der ersten Wand ein Kragen angeordnet ist, wobei der Kragen zusammen mit der Wand eine gemeinsame obere Fläche aufweist.

In manchen Ausführungsformen kennzeichnet das Ausatemsystem, dass zumindest eine Aussparung in der oberen Fläche der ersten Wand und des Kragens ausgebildet ist, wobei sich die Aussparung von einer Außenkante des Kragens bis zur Innenfläche der ersten Wand erstreckt.

In manchen Ausführungsformen kennzeichnet das Ausatemsystem, dass zumindest eine Aussparung und/oder Spalt oberhalb zumindest einer Rillenanordnung angeordnet ist und der Spalt gasleitend mit den Rillen verbunden ist.

In manchen Ausführungsformen kennzeichnet das Ausatemsystem, dass die Rillen innerhalb einer Rillenanordnung mit gleichmäßigem Abstand angeordnet sind.

In manchen Ausführungsformen kennzeichnet das Ausatemsystem, dass eine Rillenanordnung höchstens 8 Rillen umfasst.

In manchen Ausführungsformen kennzeichnet das Ausatemsystem, dass die Zahl der Rillen je Rillenanordnung unabhängig voneinander ist.

In manchen Ausführungsformen kennzeichnet das Ausatemsystem, dass in dem Ausatemsystem mindestens 12 und maximal 64 Rillen angeordnet sind.

In manchen Ausführungsformen kennzeichnet das Ausatemsystem, dass die zweite Wand im Wesentlichen rohrförmig ausgebildet ist, wobei die Innenfläche der zweiten Wand einen kreisförmigen Querschnitt aufweist.

In manchen Ausführungsformen kennzeichnet das Ausatemsystem, dass die Innenfläche der zweiten Wand einen Kugelausschnitt darstellt.

In manchen Ausführungsformen kennzeichnet das Ausatemsystem, dass die erste Wand im Wesentlichen rohrförmig ausgebildet ist.

In manchen Ausführungsformen kennzeichnet das Ausatemsystem, dass die Innenfläche der ersten Wand und die Außenfläche der zweiten Wand parallel zueinander verlaufen.

In manchen Ausführungsformen kennzeichnet das Ausatemsystem, dass die Innenfläche der ersten Wand und die Außenfläche der zweiten Wand konisch zusammenlaufen.

In manchen Ausführungsformen kennzeichnet das Ausatemsystem, dass sowohl die Innenfläche der ersten Wand als auch die Außenfläche der zweiten Wand zumindest abschnittsweise einen im Wesentlichen kreisförmigen, ovalen und/oder polygonen Querschnitt aufweisen.

In manchen Ausführungsformen kennzeichnet das Ausatemsystem, dass die zweite Wand Teil eines Verschlusses ist.

In manchen Ausführungsformen kennzeichnet das Ausatemsystem, dass die zweite Wand Teil des Maskenkörpers ist.

In manchen Ausführungsformen kennzeichnet das Ausatemsystem, dass die erste Wand über den Kragen mit dem Maskenkörper verbunden ist.

In manchen Ausführungsformen kennzeichnet das Ausatemsystem, dass die erste Wand zusammen mit dem Kragen eine Trichterform bildet, wobei die obere Fläche in einem Winkel A zu der Innenfläche steht, wobei der Winkel A zwischen 0° und 90° liegt, bevorzugt in einem Bereich von 22° bis 75°.

In manchen Ausführungsformen kennzeichnet das Ausatemsystem, dass die zweite Wand zusammen mit dem Kragen eine Trichterform bildet, wobei die Unterseite des Kragens in einem Winkel B zu der Außenfläche steht, wobei der Winkel B zwischen 0° und 90° liegt, bevorzugt in einem Bereich von 22° bis 75°.

In manchen Ausführungsformen kennzeichnet das Ausatemsystem, dass der Winkel A gleich dem Winkel B ist.

In manchen Ausführungsformen kennzeichnet das Ausatemsystem, dass die Anzahl an Aussparungen zumindest der Hälfte der Anzahl der Rillenanordnungen entspricht, maximal jedoch eins mehr als die Anzahl der Rillenanordnungen.

In manchen Ausführungsformen kennzeichnet das Ausatemsystem, dass die Rillen einen im Wesentlichen rechteckigen Querschnitt aufweisen, optional mit abgerundeten Ecken und/oder einem aufgesetzten Teilkreis.

In manchen Ausführungsformen kennzeichnet das Ausatemsystem, dass die Rillen eine maximale Breite von 1,25 mm und eine maximal Tiefe von 1,25 mm aufweisen.

In manchen Ausführungsformen kennzeichnet das Ausatemsystem, dass in der Innenfläche eine Einkerbung angeordnet ist, wobei an dem Kragen und der oberen Fläche an der Stelle der Einkerbung keine Aussparung ausgebildet ist und die Einkerbung keine gasleitende Verbindung zwischen einem Innenraum der Patientenschnittstelle und einem Außenbereich darstellt.

In manchen Ausführungsformen kennzeichnet das Ausatemsystem, dass zumindest abschnittsweise eine Griffwand auf der Oberseite des Kragens angeordnet ist.

In manchen Ausführungsformen kennzeichnet das Ausatemsystem, dass an dem Kragen zumindest ein Vorsprung angeordnet ist, wobei an dem Vorsprung eine Nase angeordnet ist. In manchen Ausführungsformen kennzeichnet das Ausatemsystem, dass an der Innenfläche der ersten Wand zumindest drei Verschlusszapfen angeordnet sind und in der Außenfläche der zweiten Wand eine entsprechende Anzahl an Verschlussschlitzen angeordnet sind.

In manchen Ausführungsformen kennzeichnet das Ausatemsystem, dass die Verschlusszapfen der ersten Wand unregelmäßig angeordnet und die Verschlussschlitze in der zweiten Wand im gleichen Maße unregelmäßig angeordnet sind, sodass die Position der Verschlussschlitze und der Verschlusszapfen in nur einer Stellung der ersten Wand zur zweiten Wand übereinstimmen.

Die Patientenschnittstelle umfasst ein Ausatemsystem nach zumindest einer der vorhergehenden Ausführungsformen und ein Kugelgelenk.

Das Kugelgelenk für eine Patientenschnittstelle umfasst zumindest einen Gelenkkopf und ein Gelenklager und wird dadurch gekennzeichnet, dass der Gelenkkopf im Wesentlichen durch die dritte Wand dargestellt wird und das Gelenklager im Wesentlichen durch die Innenfläche der zweiten Wand gebildet wird.

In manchen Ausführungsformen wird das Kugelgelenk dadurch gekennzeichnet, dass die Innenfläche der zweiten Wand kugelförmig ausgebildet ist und die Außenfläche der dritten Wand dazu komplementär kugelförmig ausgebildet ist, wobei die Krümmungsradien der Außenfläche und der Innenfläche im Wesentlichen gleich sind und/oder geringfügig (<1%) voneinander abweichen

In manchen Ausführungsformen wird das Kugelgelenk dadurch gekennzeichnet, dass die Krümmungsradien der Innenfläche der zweiten Wand und der Außenfläche der dritten Wand so gewählt sind, dass eine Bewegung der dritten Wand relativ zur zweiten Wand möglich ist.

Es ist darauf hinzuweisen, dass eine hierin verwendete, zwischen zwei Merkmalen stehende und diese miteinander verknüpfende Konjunktion "und/oder" stets so auszulegen ist, dass in einer ersten Ausgestaltung des Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiPAP-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können.

Als Patientenschnittstelle kann, soweit nicht ausdrücklich anders beschrieben, jegliches Peripheriegerät verstanden werden, welches zur Interaktion, insbesondere zu Therapie- oder Diagnosezwecken, der Messeinrichtung mit einem Lebewesen gedacht ist. Insbesondere kann eine Patientenschnittstelle als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben bzw. -kanülen und sogenannte Nasenbrillen können als Maske beziehungsweise Patientenschnittstelle eingesetzt werden. In manchen Fällen kann das Patientenschnittstelle auch ein einfaches Mundstück, beispielsweise ein Rohr, sein, durch welches das Lebewesen zumindest ausatmet und/oder einatmet.

Im Zuge der Erfindung bedeutet, wenn ein Element in einer Fläche (Innenfläche beziehungsweise Außenfläche) angeordnet ist, dass dieses Element (beispielsweise Rillen) in der Fläche versenkt angeordnet ist. In einem beispielhaften Fall der Rillen, welche zum Beispiel in der Innenfläche einer Wand angeordnet sind, sind die Rillen so angeordnet, dass diese an der Innenfläche liegen, und in die Wand hineinragen (also versenkt sind). Dem entgegen stehen Elemente, welche auf der Fläche angeordnet sind. Diese sind so an der Fläche angeordnet, dass sie gegenüber der Wand und der Fläche erhaben sind, also abstehen.

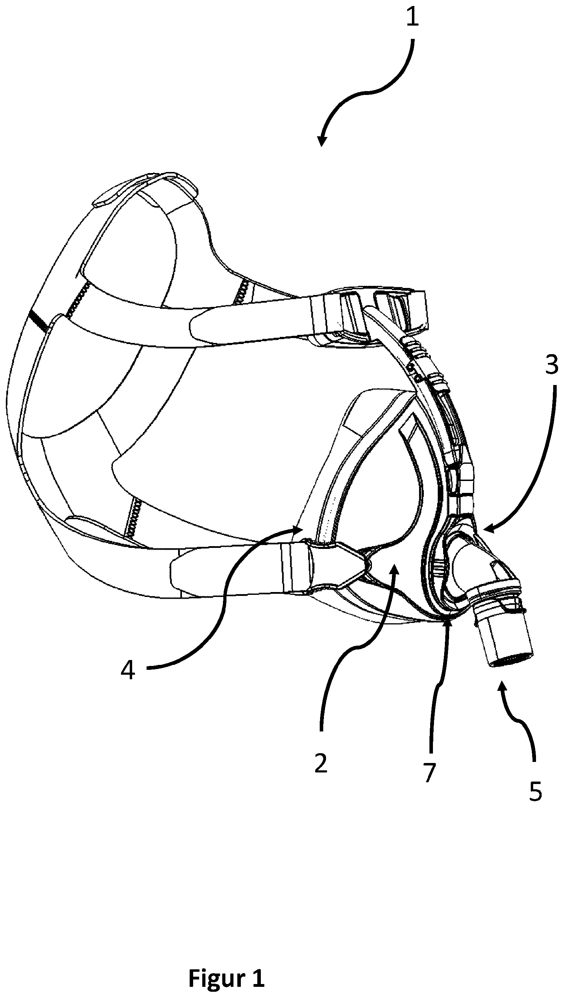

Die Erfindung wird anhand der Figuren 1 bis 12 beispielhaft näher beschrieben.

Figur 1 zeigt eine beispielhafte Ausführungsform der Patientenschnittstelle 1 als Maske mit einer beispielhaften Ausführungsform des Ausatemsystems 3. Die Patientenschnittstelle 1 umfasst beispielhaft einen Maskenkörper 2, an welchen eine Stirnstütze und eine Kopfbänderung angeordnet sind. Der Verschluss 7 bildet beispielhaft zusammen mit Teilen des Maskenkörpers 2 das Ausatemsystem 3. Der Verschluss 7 ist beispielhaft zudem so eingerichtet, dass ein Gelenkkopf 601 eines Kugelgelenks 6 aufgenommen werden kann. Über das Kugelgelenk 6 ist ein Schlauchanschluss 5 mit der Patientenschnittstelle 1 verbunden. Mit dem Maskenkörper 2 ist ein Maskenpolster 4 verbunden, welches dazu ausgebildet ist mit einer Fläche auf dem Gesicht eines Anwenders/Patienten aufzuliegen. Zudem umfasst das Maskenpolster 4 eine Öffnung, welche dazu ausgebildet ist zumindest Teile des Gesichts, wie zum Beispiel Nase und/oder Mund, aufzunehmen. Der Maskenkörper 2 und das Maskenpolster 4 umschließen zusammen einen Innenraum 101 (im Folgenden vereinfacht Innenraum 101 der Patientenschnittstelle 1), in welchen das Gesicht des Anwenders/Patienten bei der Anwendung der Maske auf dem Gesicht zumindest teilweise hereinragt. Das Maskenpolster 4 ist beispielsweise über einen Polsteranschluss 204 mit dem Maskenkörper 2 verbunden. In manchen Ausführungsformen ist das Maskenpolster 4 auch Teil des Maskenkörpers. Der Innenraum 101 der Patientenschnittstelle 1 ist über das Ausatemsystem 3 mit dem Außenbereich 102 der Patientenschnittstelle 1 gasleitend verbunden. Über den Schlauchanschluss 5 kann die Patientenschnittstelle 1 beispielsweise über einen Beatmungsschlauch mit einem Beatmungsgerät gasleitend verbunden werden. Über den Schlauchanschluss 5 kann so zum Beispiel Atemgas in und/oder aus dem Innenraum 101 der Patientenschnittstelle 1 und dem Anwender/Patienten geleitet bzw. gefördert werden.

Beispielsweise ist die Patientenschnittstelle 1 als Full-Face Maske ausgeführt, sodass von dem Maskenpolster 4 zumindest Nase und Mund des Anwenders umschlossen werden. Das Ausatemsystem 3 und das Kugelgelenk 6 kann aber auch in anderen Patientenschnittstellen 1, wie beispielsweise Nasal-Masken, Trachealkanülen/Trachealtuben und/oder Nasenbrillen Anwendung finden.

Beispielsweise wird durch den Schlauchanschluss 5 und das Kugelgelenk 6 Atemgas von einem Beatmungsgerät in den Innenraum 101 der Patientenschnittstelle 1 gefördert um den Anwender/Patienten bei der Atmung zu unterstützen. Die Förderung des Atemgases wird dazu beispielsweise über den Druck oder den Fluss kontrolliert. Durch das Ausatemsystem 3 kann gewollt ständig Atemgas entweichen, wodurch auch ausgeatmetes Atemgas des Patienten/Anwenders aus dem Innenraum 101 der Patientenschnittstelle 1 gefördert bzw. ausgewaschen wird. So wird eine Anreicherung von CO2 in dem Atemgas bzw. in dem Innenraum 101 der Patientenschnittstelle 1 verhindert und der Patienten/Anwender kann frisches Atemgas einatmen.

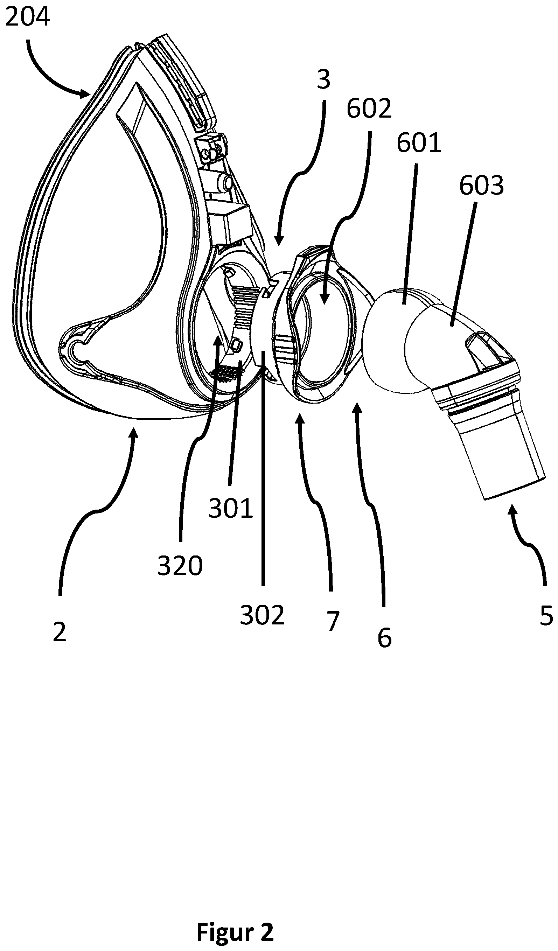

Eine Explosionsansicht des Maskenkörpers 2 zusammen mit Ausatemsystem 3 und Kugelgelenk 6 ist beispielhaft in Figur 2 dargestellt. Das Ausatemsystem 3 umfasst beispielhaft zumindest eine erste Wand 301 und eine zweite Wand 302. Die erste Wand 301 ist beispielhaft als Teil des Maskenkörpers 2 ausgeführt und umschließt eine Öffnung 320. Die Öffnung 320 ist so eingerichtet und ausgebildet, dass durch die Öffnung 320 Atemgas zum Patienten hin und/oder vom Patienten weg gefördert werden kann. Die Öffnung 320 liegt dem Polsteranschluss 204 des Maskenkörpers 2 bzw. dem Maskenpolster 4 im Wesentlichen gegenüber. Beispielhaft weißt die Öffnung 320, und damit zumindest die Innenfläche 301a der erste Wand 301 einen runden Querschnitt auf. In manchen Ausführungsformen kann die Öffnung 320 auch einen ovalen und mehreckigen, optional mit abgerundeten Ecken, Querschnitt aufweisen. Auch eine Freiform ist als Querschnitt für die Öffnung 320 denkbar. Die zweite Wand 302 ist beispielhaft als Teil des Verschlusses 7 ausgeführt. Die zweite Wand 302 neben der ersten Wand 301 an der Innenfläche 301a angeordnet ist.

In manchen Ausführungsformen kann die erste Wand 301 auch als extra Teil ausgeführt sein und in den Maskenkörper 2 eingesetzt und/oder mit dem Maskenkörper 2 verbunden werden, beispielsweise über umspritzen/umgießen, verkleben, verschweißen und/oder über lösbare Verbindungsmethoden wie zum Beispiel Stecken und/oder Schrauben.

Der Verschluss 7 weist weiter ein Gelenklager 602 auf, welche dazu ausgebildet ist, einen Gelenkkopf 601 aufzunehmen. Beispielhaft ist das Gelenklanger 602 als eine hohle Teilkugel ausgeführt, wobei die Teilkugel zumindest einen Äquator C aufweist. Das Gelenklager 602 wird beispielhaft von der Innenfläche 302a der zweiten Wand 302 gebildet. Der Gelenkkopf 601 ist beispielhaft zumindest als Teilkugel ausgeführt, wobei der Gelenkkopf 601 in manchen Ausführungsformen auch eine ganze Kugel beschreiben kann. Ist der Gelenkkopf 601 als Teilkugel ausgeführt, so weist auch der Gelenkkopf 601 zumindest einen Äquator C auf. An den Gelenkkopf 601 ist beispielhaft ein Rohrstück 603 angeordnet, welches dazu ausgebildet ist mit einem Schlauchanschluss 5 oder direkt mit einem (Beatmungs-)Schlauch verbunden zu werden. In dem Gelenkkopf 601 ist zumindest eine Durchführung ausgebildet, beispielsweise als Bohrung, damit Atemgas durch den Gelenkkopf 601 geleitet werden kann. In manchen Ausführungsformen ist der Gelenkkopf als (bzw. als Teil einer) Hohlkugel ausgeführt. Die Wandstärke des Gelenkkopfs 601 kann beispielsweise auch variabel ausgeführt sein, beispielsweise zu den Öffnungen der Durchführung hin verjüngend und im Bereich des Äquators C mit höherer Wandstärke.

Beispielhaft sind in der ersten Wand 301 Rillenanordnungen 303 angeordnet, welche je zumindest zwei Rillen 304, bevorzugt vier bis acht Rillen 304, umfassen und zusammen mit der zweiten Wand 302 Kanäle 305 bilden, welche den Innenraum 101 der Patientenschnittstelle 1 mit dem Außenbereich 102 der Patientenschnittstelle 1 verbinden Die Rillen 304 sind dabei so in der ersten Wand 301 und/oder zweiten Wand 302 angeordnet, dass diese die Innenfläche (301a, 302a) beziehungsweise Außenfläche (301b, 302b) zumindest teilweise unterbrechen.

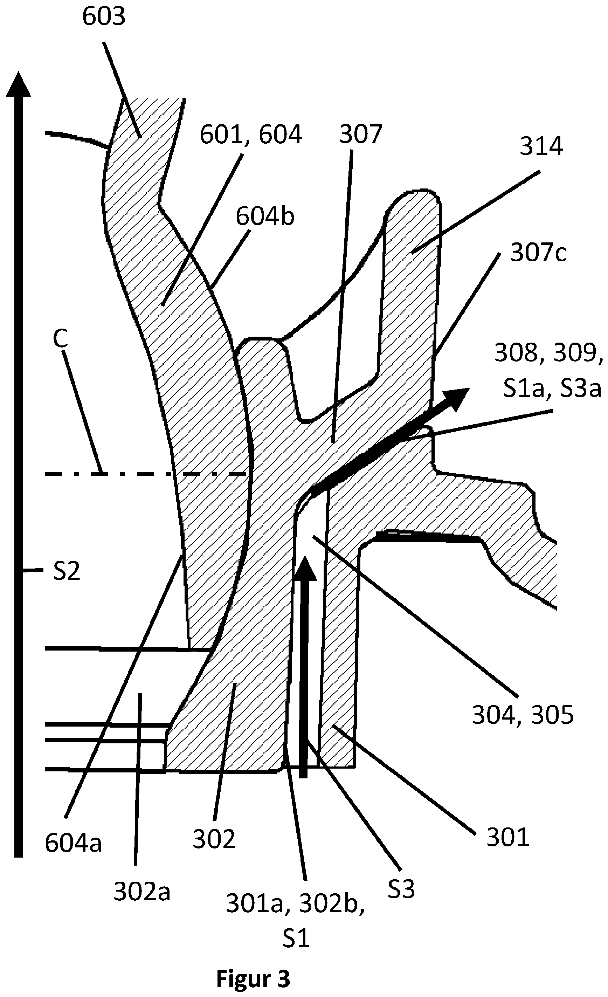

Figur 3 zeigt einen Schnitt durch das Ausatemsystem 3, bestehend aus der ersten Wand 301, der zweiten Wand 302 sowie der dritten Wand 303. Die zweite Wand 302 ist beispielhaft Teil des Verschlusses 7. Die dritte Wand 604 stellt im Wesentlichen zugleich den Gelenkkopf 601 des Kugelgelenks 6 dar. Die Wand 604 ist beispielhaft im Wesentlichen kugelschalenförmig ausgebildet. In manchen Ausführungsformen ist zumindest die Außenfläche 604b im Wesentlichen kugelförmig ausgebildet. In manchen Ausführungsformen ist die Innenfläche 604a der dritten Wand 604 im Wesentlichen rohrförmig ausgebildet und/oder weist zumindest abschnittsweise eine Kugelform auf.

An dem Gelenkkopf 601 beziehungsweise der dritten Wand 604 ist beispielhaft ein Rohrstück 603 angeordnet, welches beispielsweise mit einer Schlauchanschluss 5 oder direkt mit einem Schlauch verbunden werden kann

Die Innenfläche 302a der zweiten Wand 302 ist beispielhaft, zumindest abschnittsweise, zu dem Gelenkkopf 601 beziehungsweise der Außenfläche 604b komplementär, im Wesentlichen kugelförmig ausgebildet. Die Innenfläche 302a bildet zumindest teilweise das Gelenklager 602. Beispielhaft sind die Krümmungsradien der kugelförmigen Abschnitte der Innenfläche 302a der zweiten Wand und der Außenfläche 604b der dritten Wand 604 im Wesentlichen gleich und/oder weichen nur geringfügig (<1%) voneinander ab.

Die Krümmungsradien der Innenfläche 302a der zweiten Wand 302 und der Außenfläche 604b der dritten Wand 604 sind beispielhaft so gewählt, dass eine Bewegung der dritten Wand 604 relativ zur zweiten Wand 302 möglich ist.

Die erste Wand 301 ist neben der zweiten Wand 302 angeordnet, sodass die Innenfläche 301a der ersten Wand 301 zumindest stellenweise an der Außenfläche 302b der zweiten Wand 302 anliegt. Die Außenfläche 302b verläuft im Wesentlichen parallel zu der Innenfläche 301a. Zumindest abschnittsweise ist zwischen der Innenfläche 301a und der Außenfläche 302b ein Spalt ausgebildet, durch welchen ein erster Strömungsweg S1 verläuft, welcher im Wesentlichen eine Strömung von Atemgas von dem Innenraum 101 der Patientenschnittstelle 1 in einen Außenbereich 102 ermöglicht und in einem gewissen Maße auch zumindest zeitweise einen Abbau von Atemgasdruck im Innenraum 101 dient. Der Spalt, also der Abstand zwischen der Innenfläche 301a und der Außenfläche 302b, beträgt maximal 0,3 mm, bevorzugt ist der Abstand zwischen der Innenfläche 301a und der Außenfläche 302b maximal 0,2 mm. Abschnittsweise kann die Innenfläche 301a auch unmittelbar an der Außenfläche 302b anliegen, der Spalt wäre also <0,05mm.

In der in Figur 3 gezeigten beispielhaften Ausführungsform sind beispielsweise an der Außenfläche 301b der ersten Wand 301 ein Kragen 306 und an der Außenfläche 302b der zweiten Wand 302 ein Kragen 307 angeordnet. Die gemeinsame obere Fläche 301c der ersten Wand 301 und des Kragens 306 steht beispielhaft in einem Winkel A zu der Innenfläche 301a. Die Unterseite 307b des Kragens 307 steht beispielhaft in einem Winkel B zu der Außenfläche 307b der zweiten Wand. In der beispielhaften Ausführungsform entspricht der Winkel A dem Winkel B, wobei der Winkel A in einem Bereich zwischen 0° und 90° liegt, bevorzugt in einem Bereich von 22° bis 75°. Die Unterseite 307b liegt im Wesentlichen auf der oberen Fläche 301c auf, wobei sich zwischen Unterseite 307b und oberer Fläche 301c zumindest abschnittsweise ein Spalt bildet.

Die Unterseite 307b und die obere Fläche 301c sind in manchen Ausführungsformen so ausgebildet, dass zumindest abschnittsweise ein Spalt entsteht, welcher eine Verlängerung S1a des ersten Strömungsweges S1 darstellt. Zumindest abschnittsweise kann Atemgas zwischen der Unterseite 307b und der oberen Fläche 301c strömen.

Beispielhaft sind in der Innenfläche 301a der ersten Wand 302 Rillen 304 versenkt angeordnet. Die Rillen 304 bilden zusammen mit der Außenfläche 302b der zweiten Wand 302 Kanäle 305 aus, durch welche die Teilströmungswege S3 verlaufen. Die Teilströmungswege S3 sind beispielhaft Teil des Strömungsweges S1, wobei die Teilströmungswege S3 explizit nur durch die Kanäle 305 verlaufen. Der Strömungsweg S1 beschreibt also beispielhaft den gesamten Strömungsweg zwischen der ersten Wand 301 und der zweiten Wand 302, wobei die Teilströmungswege S3 die Teile des Strömungsweges S1 beschreiben, welche durch die Kanäle 305 verlaufen.

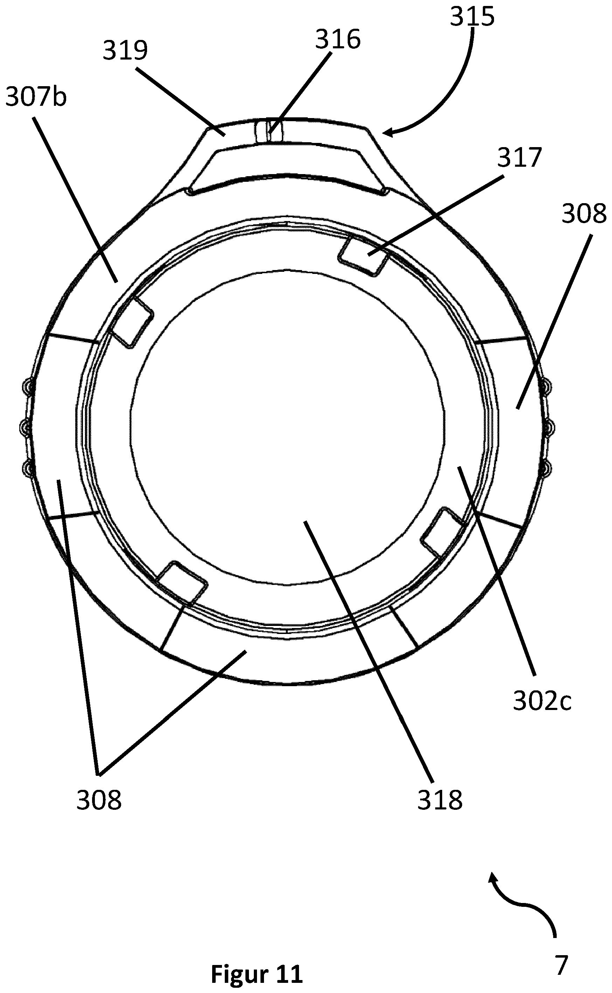

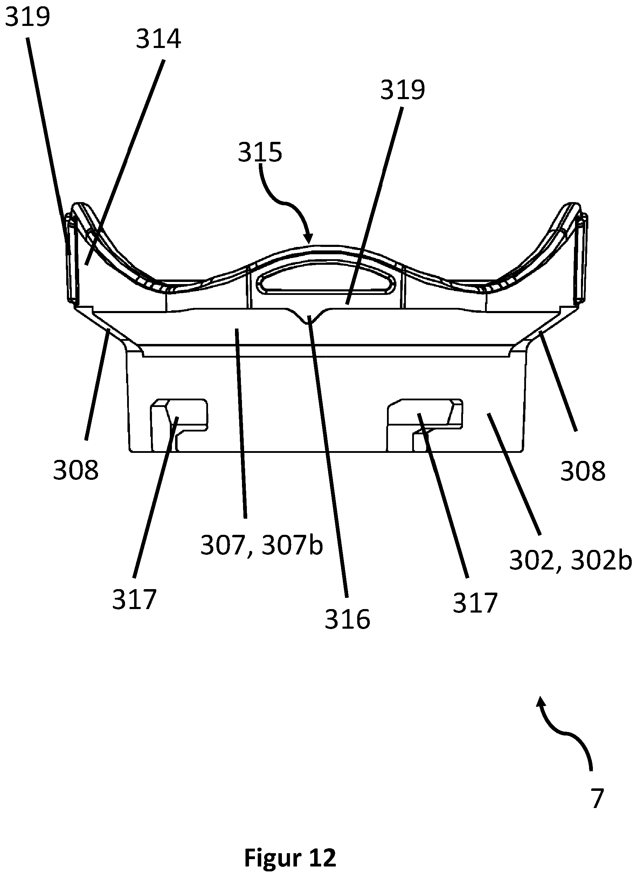

Beispielhaft sind in der Unterseite 307b des Kragens 307 Aussparungen 308 angeordnet beziehungsweise ausgebildet, welche zusammen mit der oberen Fläche 301c die Spalte 309 bilden. Durch die Spalte 309 verlaufen beispielsweise Verlängerungen S3a der Teilströmungswege S3. In manchen Ausführungsformen sind der Strömungsquerschnitt vom Teilströmungsweg S3 und der Strömungsquerschnitt der Verlängerung S3a nicht gleich. In manchen Ausführungsformen gehen zumindest zwei, maximal aber 16 Teilströmungswege S3 in eine Verlängerung S3a über, entsprechend der Anordnung von Aussparungen 308 in der Unterseite 307b des Kragens 307 (siehe auch Figuren 11 und 12). In manchen Ausführungsformen geht auch der Strömungsweg S1 zumindest Stellenweise in die Verlängerung S3a über. Die Spaltweite des Spaltes 309, beispielsweise dominiert durch die Tiefe der Aussparung 308, beträgt zwischen 0,1 mm und 0,8 mm, bevorzugt zwischen 0,25 mm und 0,45 mm. Beispielhaft liegt die Spaltweite des Spaltes 309 bei 0,35 mm. Die Breite des Spaltes 309 liegt beispielsweise in einem Bereich von 10 mm bis 30 mm. In manchen Ausführungsformen kann die Breite des Spaltes in Richtung Außenseite 307c zunehmen. In anderen Ausführungsformen kann die Breite des Spaltes aber auch konstant bleiben. Insbesondere ist die Breite des Spalte 309 von der Positionierung (Basisbereich 324, Seitenbereich 322) und auch von der Anzahl und Anordnung der Kanäle 305 abhängig. Der Spalt 309, welcher in dem Basisbereich 324 angeordnet ist, weitet sich beispielhaft von 18 mm auf 22 mm auf, die Spalte 309 in den Seitenbereichen 322 weiten sich beispielsweise von 1,5 mm auf 16 mm auf. Die Länge der Spalte 309 liegt in einem Bereich zwischen 2 mm und 10 mm, bevorzugt zwischen 3 mm und 6 mm. Beispielhaft liegt die Länge der Spalte bei 4,5 mm.

Die Strömung durch den Teilströmungsweg S3 macht den Großteil (>95%, bevorzugt >99%) der Gesamtströmung durch den Strömungsweg S1 aus, die Strömung in dem Spalt zwischen der Innenfläche 301a und der Außenfläche 302b liegt dabei bei maximal 5%, bevorzugt maximal 1%, der Gesamtströmung durch den Strömungsweg S1. Die Strömung durch die Verlängerung S1a ist im Vergleich zu der Strömung durch die Verlängerung S3a vernachlässigbar klein, beispielsweise stehen die Strömungen durch S1a und S3a in einem Verhältnis S1a:S3a von maximal 1:100, wobei das Verhältnis eher noch kleiner ist.

Die dritte Wand 604 ist neben der zweiten Wand 302 so angeordnet, dass die Außenfläche 604b zumindest stellenweise an der Innenfläche 302a anliegt. Die Innenfläche 604a der dritten Wand 604 ist zumindest abschnittsweise rohrförmig ausgebildet, sodass die Innenfläche 604a der dritten Wand 604 einen zweiten Strömungsweg S2 umgibt. Der Strömungsquerschnitt des zweiten Strömungsweges S2 liegt beispielhaft in einem Bereich von 600 mm² bis 5000 mm², bevorzugt zwischen 700 mm² bis 3500 mm², mehr bevorzugt zwischen 800 mm² und 2500 mm². In manchen Ausführungsformen variiert der Strömungsquerschnitt entlang der Innenfläche 604a, so kann der Strömungsquerschnitt beispielsweise an einer Seite/Öffnung bei etwa 800 mm² liegen und sich zur Mitte hin auf 2500 mm² aufweiten. Gegebenenfalls kann der Strömungsquerschnitt zur anderen Seite/Öffnung/Übergang zu einem Rohrstück 603 wieder verengen. Ist die Innenfläche 604a der dritten Wand 604 kreisförmig ausgebildet, so kann der Durchmesser in einem Bereich von 14 mm bis 40 mm, bevorzugt von 15 mm bis 30 mm, liegen. Der Strömungsweg S2 ermöglicht zumindest zeitweise die Strömung von Atemgas aus dem Innenraum 101 der Patientenschnittstelle 1. Beispielsweise ist zumindest zeitweise eine Strömung von Atemgas über den Strömungsweg S2 aus dem Innenraum 101 zum Beispiel in einen angeschlossenen Schlauch möglich. Ist an dem Rohrstück 603 beispielsweise ein Schlauch - zum Beispiel über einen Schlauchanschluss 5 - angeschlossen, führt der zweite Strömungsweg S2 zumindest teilweise in den angeschlossenen Schlauch. In manchen Ausführungsformen kann über das Rohrstück 603, zusammen mit einem Schlauch und gegebenenfalls einem Schlauchanschluss 5, ein Beatmungsgerät mit der Patientenschnittstelle 1 verbunden werden, sodass entgegen des Strömungsweges S2 auch Atemgas in den Innenraum 101 der Patientenschnittstelle 1 strömen kann bzw. durch das Beatmungsgerät gefördert wird.

In manchen Ausführungsformen weist der zweite Strömungsweg S2 einen Abzweig S2a auf, welcher durch den Spalt zwischen der Außenfläche 604b und der Innenfläche 302a verläuft. Der Abzweig S2a verläuft zumindest stellenweise koaxial um den zentral verlaufenden Strömungsweg S2. Der Strömungsweg S2 wird in manchen Ausführungsformen zumindest stellenweise auch von der Innenfläche 302a der zweiten Wand 302 umgeben. Der Spalt zwischen der Außenfläche 604b und der Innenfläche 302a weist zumindest zeitweise und zumindest stellenweise eine Weite in einem Bereich von 0,01 mm und 0,3 mm auf, bevorzugt zwischen 0,05 mm und 0,22 mm. In manchen Ausführungsformen kann zumindest zeitweise Atemgas durch den Abzweig S2a aus dem Innenraum 101 strömen. In manchen Ausführungsformen wird die Außenfläche 604b zumindest stellenweise an die Innenfläche 302a gedrückt, sobald sich ein gewisser Überdruck im Innenraum 101 aufbaut. In manchen Ausführungsformen wird der Spalt zwischen Außenfläche 604b und der Innenfläche 302a bei Anliegen eines gewissen Überdruckes verschlossen. In manchen Ausführungsformen verringert sich die Spaltweite zwischen der Außenfläche 604b und der Innenfläche 302a dadurch soweit, dass eine nur sehr geringe Strömung durch den Abzweig S2a möglich ist. In manchen Ausführungsformen bildet sich durch die Strömung zwischen Außenfläche 604b und der Innenfläche 302a ein gewisses Luftpolster bildet, welches beispielsweise eine vereinfachte Bewegung der Wand 604 relativ zur Wand 302 ermöglicht.

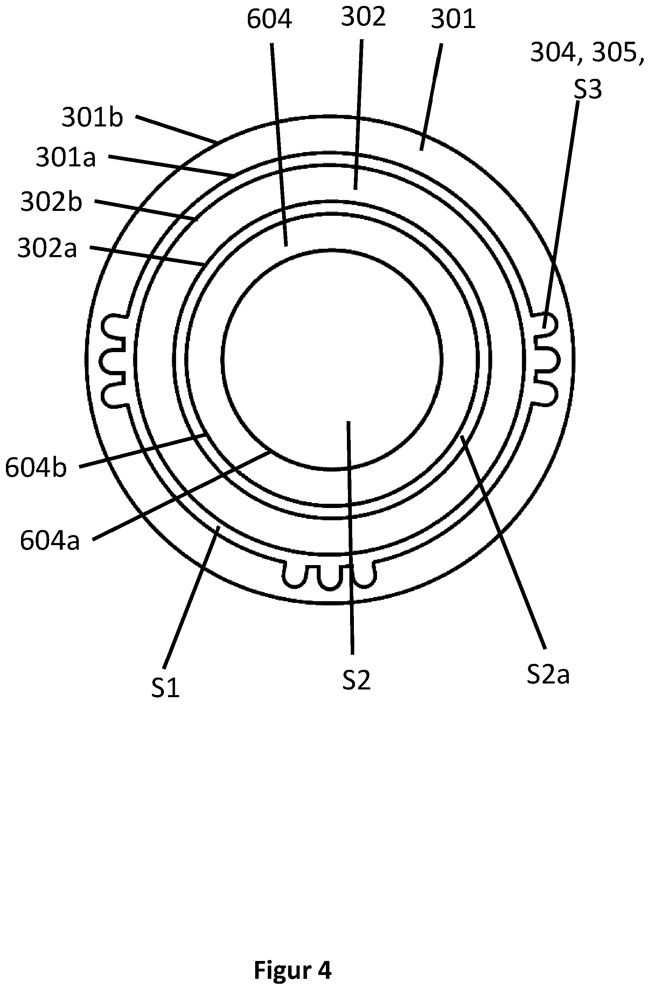

Figur 4 zeigt einen stark vereinfachten, schematischen Schnitt durch eine beispielhafte Ausführungsform des Ausatemsystems 3. Anhand dieser Figur soll der Verlauf der einzelnen Strömungswege sowie der Wände zueinander verdeutlicht werden.

Der erste Strömungsweg S1 verläuft zwischen der ersten Wand 301 und der zweiten Wand 302. Die erste Wand 301 und die zweite Wand 302 sind so angeordnet, dass zumindest stellenweise ein Spalt zwischen der Innenfläche 301a der ersten Wand 301 und der Außenfläche 302b der zweiten Wand 302 entsteht. Der erste Strömungsweg S1 verläuft zumindest teilweise in dem Spalt zwischen der Innenfläche 301a der ersten Wand 301 und der Außenfläche 302b der zweiten Wand 302. In der Innenfläche 301a der ersten Wand 301 sind Rillen 304 versenkt angeordnet, welche zusammen mit der Außenfläche 302b der zweiten Wand 302 die Kanäle 305 ausbilden. Durch die Kanäle 305 verläuft ein Teil des Strömungsweges S1, der Teilströmungsweg S3. Im Vergleich zum Spalt zwischen der Innenfläche 301a und der Außenfläche 302b, ist der Strömungsquerschnitt des Teilströmungsweges S3 deutlich größer. Insgesamt macht der Teilströmungsweg S3 also einen Großteil (>95%, bevorzugt >99%) des gesamten Strömungsquerschnitts des ersten Strömungsweges S1 aus.

Der zweite Strömungsweg S2 verläuft im Wesentlichen zentral und ist zumindest abschnittsweise von der Innenfläche 604a der dritten Wand 604 umgeben. Die Innenfläche 604a ist beispielhaft rohrförmig ausgeführt, wobei der Querschnitt entlang der Innenfläche 604a variieren kann.

Die zweite Wand 302 und die dritte Wand 604 sind beispielhaft so angeordnet, dass zwischen der Außenfläche 604b der dritten Wand 604 und der Innenfläche 302a der zweiten Wand 302 zumindest stellenweise ein Spalt entsteht, welcher eine Bewegung der dritten Wand 604 relativ zur zweiten Wand 302 ermöglicht. Ein Abzweig S2a des zweiten Strömungsweges S2 verläuft durch den Spalt zwischen der Außenfläche 604b und der Innenfläche 302a, wobei der Strömungsquerschnitt des Abzweiges S2a ein Bruchteil (<1%) des zweiten Strömungsweges S2 umfasst.

Beispielhaft verläuft der Abzweig S2a zumindest abschnittsweise koaxial um den zentral verlaufenden zweiten Strömungsweg S2. Der erste Strömungsweg S1 verläuft zumindest abschnittsweise koaxial um den Abzweig S2a. Die Teilströmungswege S3 durch die Kanäle 305 verlaufen zumindest teilweise parallel zu dem Strömungsweg S1 zwischen der ersten Wand 301 und der zweiten Wand 302.

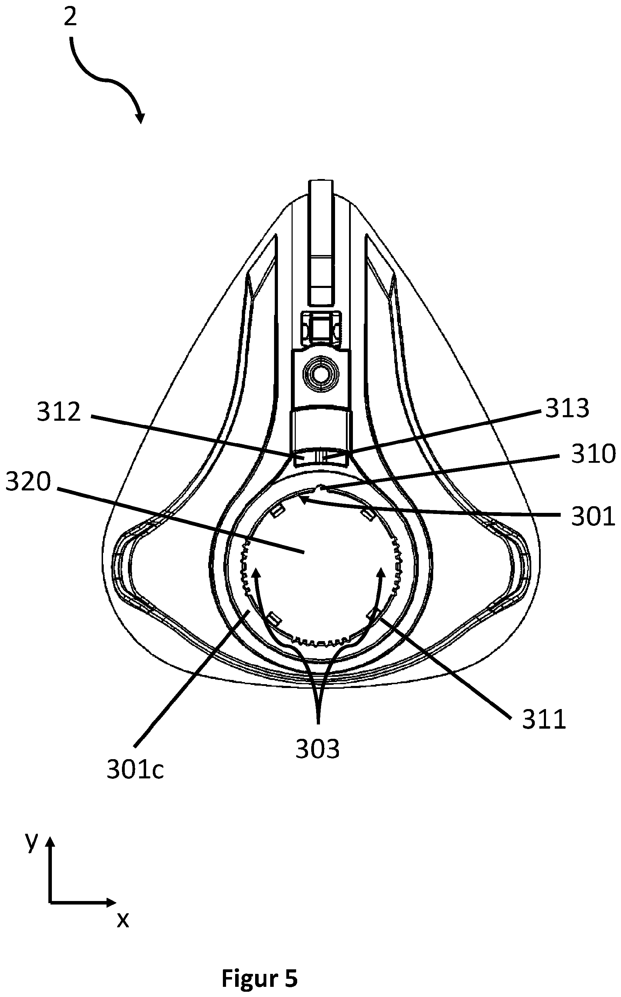

Figur 5 zeigt eine beispielhafte Ausführungsform des Maskenkörpers 2 mit der ersten Wand 301 des Ausatemsystems 3 in Frontansicht. Die erste Wand 301 umgibt die Öffnung 320. Beispielhaft sind in der Innenfläche 301a der ersten Wand 301 Rillenanordnungen 303 angeordnet beziehungsweise versenkt angeordnet. In der gezeigten, beispielhaften Ausführungsform sind insgesamt sechs Rillenanordnungen 303 angeordnet, wobei die Rillenanordnungen 303 jeweils beispielhaft vier Rillen 304 umfassen. Die Rillenanordnungen 303 sind jeweils paarweise angeordnet. Der Abstand zwischen den Rillenanordnungen 303 innerhalb eines Paars ist dabei kleiner als der Abstand zweier Paare zueinander. Der Abstand zwischen den Rillen 304 innerhalb einer Rillenanordnung ist beispielhaft geringer als der Abstand zwischen benachbarten Rillen 304 zweier Rillenanordnungen 303 innerhalb eines Paares. In manchen nicht erfindungsgemäßen Ausführungsformen sind in der Innenfläche 301a der ersten Wand 301 drei Rillenanordnungen 303 angeordnet, wobei die Rillenanordnungen 303 zwei bis acht Rillen 304 umfassen. Innerhalb einer Rillenanordnung 303 können die Abstände zwischen den Rillen 304 variieren. Bevorzugt sind die Abstände zwischen den Rillen 304 innerhalb einer Rillenanordnung 303 gleichmäßig und/oder variieren nur geringfügig (+/- 10%). Vorzugsweise sind die Rillenanordnungen 303 nicht gleichmäßig um die Öffnung 320 verteilt.

Beispielhaft ist an der Position in der ersten Wand 301, in welche keine Rillen 304 vorgesehen sind, eine Einkerbung 310 ausgebildet. Diese Einkerbung 310 dient nicht primär dazu, einen Kanal 305 zwischen Innenraum 101 und Außenbereich 102 der Patientenschnittstelle 1 zu bilden, sondern ist für die Fertigung des Maskenkörpers 2 in Spritzgusstechnik ausgebildet. In manchen Ausführungsformen bildet die Einkerbung 310 zusammen mit der zweiten Wand 302 zwar einen Kanal, es ist aber keine Aussparung 308 im Kragen 307 des Verschlusses 7 (siehe Figuren 11 und 12) vorgesehen um einen erhöhten Strömungsquerschnitt zu erreichen. Beispielhaft ist hier ein zentraler Punkt zum Einspritzen des Materials, wodurch bei der Entformung ein Grat entstehen kann. Um zu verhindern, dass dieser Grat auf der Innenfläche 301a der ersten Wand 301 liegt und die Anordnung der zweiten Wand 302 stört, ist in der ersten Wand 301 eine Einkerbung 310 angeordnet.

Oberhalb (in y-Richtung der Figur 5) der Öffnung 320 und außerhalb der oberen Fläche 301c der ersten Wand 301 ist beispielhaft eine Vertiefung 312 angeordnet. Innerhalb der Vertiefung 312 ist beispielhaft mittig eine Erhebung 313 ausgebildet. Die Erhebung 313 ist beispielsweise vom Boden der Vertiefung 312 nach oben zulaufend, besitzt also eine breitere Basis und verjüngt sich zu einem Scheitelpunkt. Durch diese Anordnung kann beispielsweise über eine komplementäre Nase 316 am Verschluss 7 (siehe z.B. Figur 12) ein Verschlussmechanismus bereitgestellt werden, welcher eine Drehbewegung des Verschlusses 7 innerhalb der Öffnung 320 zumindest soweit erschwert, dass ein versehentliches Drehen des Verschlusses 7 verhindert wird.

An der Innenfläche 301a sind beispielhaft vier Verschlusszapfen 311 angeordnet, welche zumindest teilweise in die Öffnung 320 hineinragen. Zusammen mit Verschlussschlitzen 317, welche in entsprechender Weise angeordnet sind, in der zweiten Wand 302 (siehe Figur 11) wird so ein Bajonett-Verschluss zwischen der ersten Wand 301 und der zweiten Wand 302 realisiert, sodass nach einrasten der Nase 316 hinter der Erhebung 313 der Verschluss 7 gegen (versehentliches) Lösen aus der Öffnung 320 gesichert ist. Die Verschlusszapfen 311 sind dabei beispielhaft in unregelmäßigen Abständen voneinander angeordnet, sodass der Verschluss 7 nur in einer Position in die Öffnung 320 geführt werden kann. Dadurch wird beispielsweise gewährleistet, dass der Verschluss 7 immer in der richtigen Orientierung eingesetzt wird und die Kanäle 305 nicht durch den Kragen 307 des Verschlusses 7 verschlossen werden oder nur teilweise unter den Aussparungen 308 liegen. In manchen Ausführungsformen können die Verschlusszapfen 311 auch mit regelmäßigen Abständen angeordnet sein oder in einer Art und Weise, dass der Verschluss beispielsweise in zwei Orientierungen eingesetzt werden kann. Auch ist daran gedacht, dass beispielsweise nur drei oder fünf und mehr Verschlusszapfen 311 an der Innenfläche 301a der ersten Wand angeordnet sein können. In manchen Ausführungsformen sind die Verschlusszapfen 311 an der zweiten Wand 302 angeordnet und die Verschlussschlitze 317 befinden sich in der ersten Wand 301. Darüber hinaus sind auch andere Verschlussarten denkbar. Beispielsweise kann es auch realisiert werden, dass sowohl die erste Wand 301 als auch die zweite Wand 302 ein Schraubgewinde aufweisen und der Verschluss 7 in die Öffnung 320 eingeschraubt wird. Auch einfache Steckverbindungen sind möglich. In manchen Ausführungsformen ist es auch denkbar, dass der Verschluss 7 beispielsweise durch einen Bügelverschluss oder Klammern in der Öffnung 320 fixiert wird.

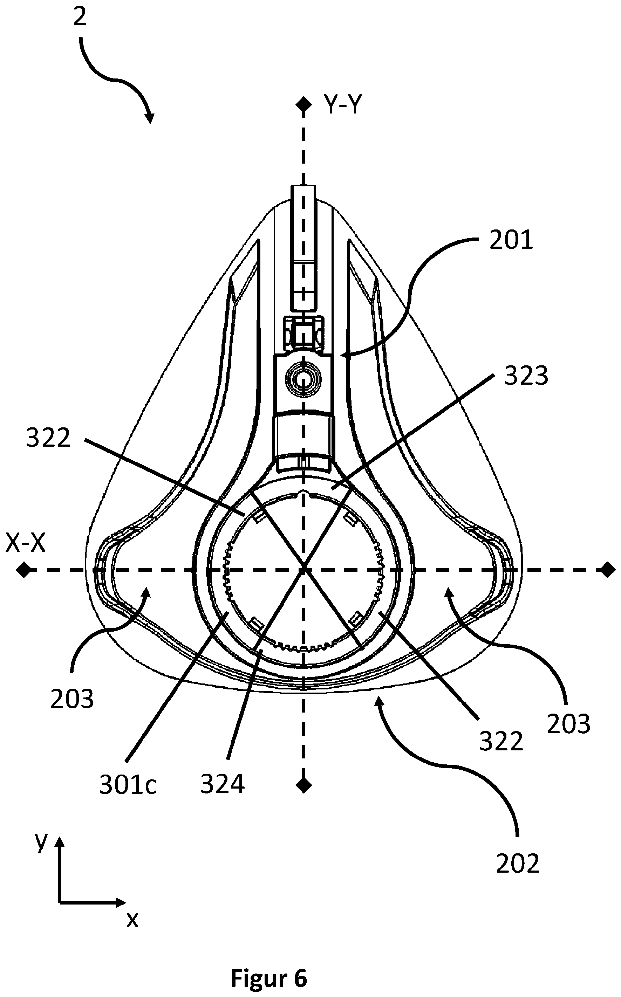

In Figur 6 sind beispielhaft vier Bereiche auf der oberen Fläche 301c der ersten Wand 301 eingezeichnet - zwei Seitenbereiche 322, ein Nasenbereich 323 und ein Basisbereich 324. Diese Bereiche entsprechen in etwa der Aufteilung der sich anschließenden Bereiche (Seitenbereich 203, Nasenbereich 201 und Basisbereich 202) des Maskenkörpers 2. Beispielhaft sind die Rillenanordnungen 303 nur in den zwei Seitenbereichen 322 und/oder dem Basisbereich 324 der ersten Wand 301 angeordnet. Der Nasenbereich 323, in welchem beispielhaft keine Rillenanordnung 303 vorgesehen ist, nimmt dabei maximal einen Bereich von 120° - im Falle einer kreisförmigen Öffnung 320 - ein. In manchen Ausführungsformen ist der Nasenbereich 323 kleiner, beispielsweise maximal 90°. In manchen Ausführungsformen des Ausatemsystems 3 sind in allen Bereichen 322, 323 und 324 Rillenanordnungen 303 angeordnet. In manchen Ausführungsformen können Rillen entlang des gesamten Umfangs der Öffnung 320 in der Innenfläche 301a der ersten Wand angeordnet sein.

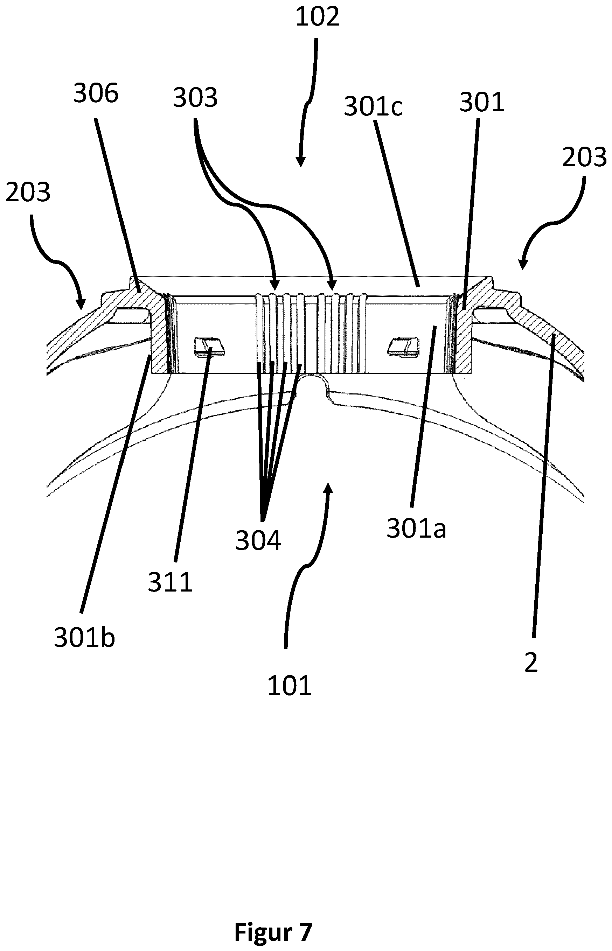

In Figur 7 ist ein Schnitt entlang der Schnittkante X-X (Figur 6) durch eine beispielhafte Ausführungsform des Maskenkörpers 2 mit Blick auf die Rillenanordnungen 303 im Basisbereich 324 der ersten Wand 301.

Auf der Innenfläche 301a der ersten Wand 301 sind Verschlusszapfen 311 angeordnet, welche beispielsweise so eingerichtet und ausgebildet sind zusammen mit Verschlussschlitzen 317 in der zweiten Wand 302 einen Bajonett-Verschluss darzustellen. Der Bajonett-Verschluss verhindert in manchen Ausführungsformen eine translatorische Bewegung der beiden Wände 301, 302 zueinander, also, dass die zweite Wand 302 (als Teil des Verschlusses 7) unbeabsichtigt aus der Öffnung 320 rutscht/gezogen werden kann. Die versehentliche bzw. unbeabsichtigte Öffnung des Bajonett-Verschlusses durch Drehen des Verschlusses 7 in eine Position, in der die zweite Wand 302 gegenüber der ersten Wand 301 verschiebbar wird, wird beispielhaft durch ein Einrasten der Nase 316 hinter der Erhebung 313 (siehe Figuren 5 und 11) verhindert. Die Verschlusszapfen 311 sind beispielhaft so angeordnet, dass der Verschluss 7 in nur einer Position und Orientierung in die Öffnung 320 eingeführt werden kann.

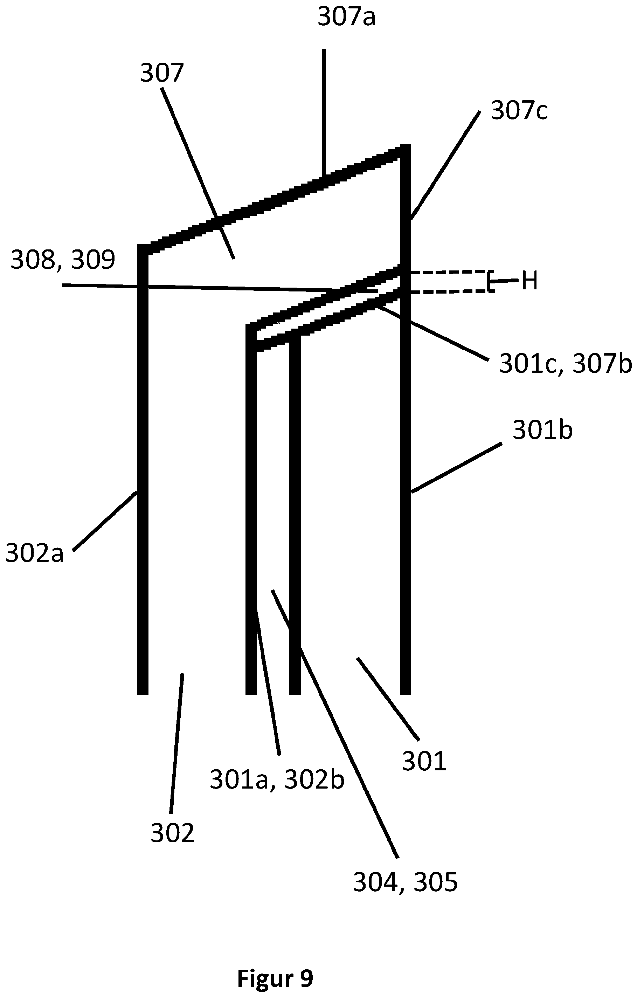

In der Innenfläche 301a der ersten Wand 301 sind die Rillenanordnungen 303 versenkt angeordnet. Beispielhaft sind drei Paare von Rillenanordnungen 303 zu je vier Rillen 304 in der Wand 301 angeordnet, wobei der Schnitt X-X aus Figur 6 durch zwei der Paare an Rillenanordnungen 304 geht. Zusammen mit der Außenfläche 302b der zweiten Wand 302 (siehe Figuren 9 bis 12) bilden die Rillen 304 Kanäle 305, welche den Innenraum 101 der Patientenschnittstelle 1 mit dem Außenbereich 102 gasleitend verbinden. Innerhalb einer Rillenanordnung 303 ist der Abstand zwischen den Rillen 304 beispielhaft gleichmäßig. Der Abstand zwischen den Rillen 304 innerhalb einer Rillenanordnung 303 ist beispielhaft kleiner als der Abstand zwischen den äußeren Rillen 304 zweier benachbarter Rillenanordnungen 303. Erfindungsgemäβ ist der Abstand zwischen zwei Paaren von Rillenanordnungen 303 größer als der Abstand der Rillenanordnungen 303 innerhalb eines Paares. In manchen nicht erfindungsgemäßen Ausführungsformen ist der Abstand aller Rillen 304 innerhalb eines Paares von Rillenanordnungen 303 gleich. In manchen nicht erfindungsgemäßen Ausführungsformen sind die Rillen 304 unabhängig von den Rillenanordnungen 303 gleichmäßig entlang der ersten Wand 301 verteilt. In manchen nicht erfindungsgemäßen Ausführungsformen sind die Rillen 304 unabhängig von den Rillenanordnungen 304 zufällig verteilt. In manchen nicht erfindungsgemäßen Ausführungsformen sind die Rillenanordnungen nicht paarweise angeordnet und in gleichmäßigen und/oder zufälligen Abständen voneinander angeordnet. In manchen Ausführungsformen sind 24 bis 64 Rillen 304 entlang der ersten Wand 301 angeordnet. In manchen Ausführungsformen sind die Abstände zwischen zwei Rillen 304 so breit wie die Rillen 304, weist eine Rille beispielsweise eine Breite von 1 mm auf, so ist der Abstand zu nächsten Rille ebenfalls 1 mm. In manchen Ausführungsformen ist der Abstand zwischen zwei Rillen 304 unabhängig von der Breite der Rillen 304.

Die Rillen 304 weisen beispielhaft eine Länge in einem Bereich zwischen 5 mm und 20 mm auf, bevorzugt zwischen 7 mm und 12 mm, beispielsweise 8 mm (+/- 5%). Die Breite der Rillen 304 an der Innenfläche 301a liegt beispielhaft in einen Bereich von 0,75 mm bis 1,25 mm, bevorzugt zwischen 0,85 mm und 1,1 mm, mehr bevorzugt zwischen 0,92 mm und 1,05 mm. Die Tiefe der Rillen liegt beispielhaft in einen Bereich von 0,75 mm bis 1,25 mm, bevorzugt zwischen 0,85 mm und 1,1 mm, mehr bevorzugt zwischen 0,92 mm und 1,05 mm. Der Strömungsquerschnitt der Kanäle 305 liegt in einem Bereich zwischen 0,7 mm² und 1,1 mm², bevorzugt zwischen 0,75 mm² und 0,9 mm², beispielsweise bei 0,8 mm² (+/- 5%). Die Breite der Rillen 304 kann in der Wand variieren, insbesondere wenn der Querschnitt der Rillen 304 nicht rechteckig ist. In manchen Ausführungsformen kann die Breite der Rillen 304 an der Innenfläche 301a variieren, beispielsweise innerhalb einer Rillenanordnung 303. Der Abstand zwischen zwei Rillen 304 innerhalb einer Rillenanordnung 303 liegt beispielhaft in einem Bereich von 0,25 mm bis 2 mm, bevorzugt zwischen 0,3 mm und 1,5 mm.

In manchen Ausführungsformen ist zwischen zwei Paaren von Rillenanordnungen 303 zumindest ein Verschlusszapfen 311 angeordnet.

In der in Figur 7 gezeigten beispielhaften Ausführungsform der ersten Wand 301 ist an der Außenfläche 301b der ersten Wand 301 ein Kragen 306 angeordnet. Der Kragen 306 ist beispielhaft so angeordnet, dass er eine gemeinsame obere Fläche 301c mit der Wand aufweist. Beispielhaft ist der Kragen 306 die Verbindung zwischen der ersten Wand 301 und dem Maskenkörper 2. In der beispielhaften Ausführungsform geht der Kragen materialmäßig in den Maskenkörper 2 über, die erste Wand 301 und der Kragen 306 sind also in den Maskenkörper 2 integriert. In manchen Ausführungsformen ist die erste Wand 301 und der Kragen 306 nicht materialmäßig mit dem Maskenkörper 2 verbunden, sondern wird als separates Teil gefertigt und in eine dafür vorgesehene Öffnung des Maskenkörpers 2 eingesetzt und mit dem Maskenkörper 2 verbunden, beispielsweise über eine Schraub- und/oder Steckverbindung, durch Verkleben und/oder durch Verschweißen. In manchen Ausführungsformen wird die Verbindung durch ein Umspritzen bzw. Umgießen erreicht. In manchen Ausführungsformen ist der Maskenkörper 2 auch direkt mit der ersten Wand 301 verbunden, beispielsweise wenn kein Kragen 306 an der ersten Wand 301 angeordnet ist. In manchen Ausführungsformen verläuft der Kragen 306 auch oberhalb von dem Maskenkörper 2, sodass ein kleiner Spalt zwischen Maskenkörper 2 und Kragen 306 entsteht.

Die obere Fläche 301c steht beispielhaft in einem Winkel A zu der Innenfläche 301a. Der Winkel A liegt dabei zwischen 0° und 90°, bevorzugt in einem Bereich von 22° bis 75°, mehr bevorzugt in einem Bereich von 35° bis 65°. In manchen Ausführungsformen liegt der Winkel A in einem Bereich von 45° bis 60°. In manchen Ausführungsformen ist die Wand 301 zusammen mit dem Kragen 306 beispielsweise trichterförmig ausgebildet, wobei die Wand 301 beziehungsweise die Innenfläche 301a den Trichterhals darstellt.

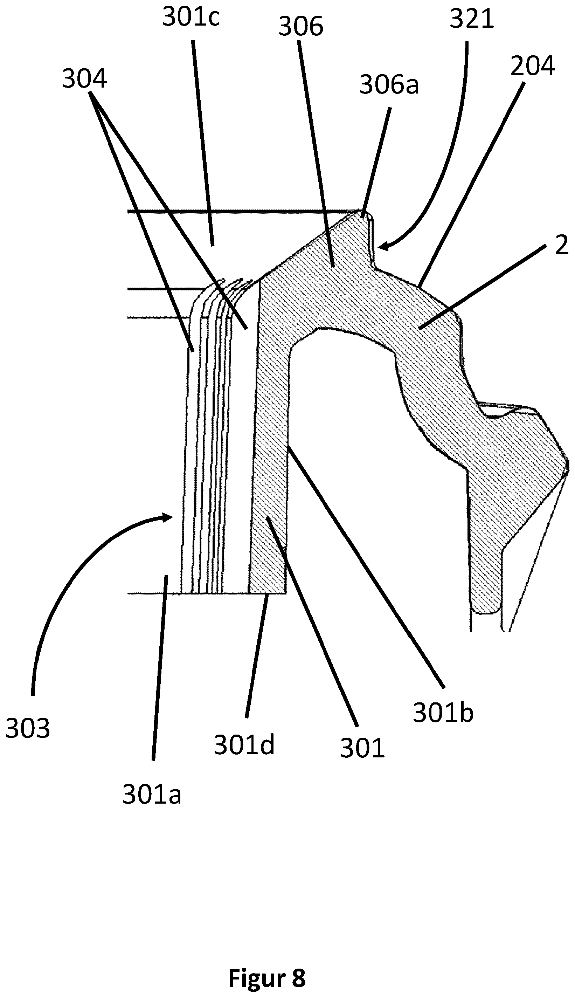

Eine beispielhafte detaillierte Ansicht der ersten Wand 301 ist in Figur 8 dargestellt. Dargestellt ist ein Schnitt durch eine Rille 304, beispielhaft entlang der Schnittkante Y-Y aus Figur 6. Die Rillen 304 sind in der Innenfläche 301a der ersten Wand 301 versenkt angeordnet und verlaufen von der unteren Fläche 301d der ersten Wand 301 bis zur oberen Fläche 301c. Zusammen mit einer zweiten Wand 302 bilde die Rillen 304 Kanäle 305, welche den Innenraum 101 der Patientenschnittstelle 1 mit dem Außenbereich 102 gasleitend verbinden. Die vier beispielhaft gezeigten Rillen 304 sind beispielsweise zusammen eine Rillenanordnung 303. An der Außenfläche 301b der ersten Wand 301 ist beispielhaft ein Kragen 306 angeordnet. In der gezeigten Ausführungsform haben die erste Wand 301 und der Kragen 306 eine gemeinsame obere Fläche 301c. Die obere Fläche 301c steht in einem Winkel A zu der Innenfläche 301a der ersten Wand 301. Beispielhaft geht der Kragen 306 materialmäßig in den Maskenkörper 2 über. In manchen Ausführungsformen ist an dem Übergang zwischen Kragen 306 und Maskenkörper 2 an der Außenkante 306a zumindest abschnittsweise eine Stufe 321 ausgebildet. Diese Stufe 321 beeinflusst dabei den Strömungsverlauf des Atemgases im Außenbereich 102 beziehungsweise an der Oberfläche 204 des Maskenkörpers 2. Der Abstand zwischen Außenkante 306a und Oberfläche 204 liegt beispielsweise in einem Bereich von 0,5 mm bis 4mm, bevorzugt in einem Bereich von 0,7 mm bis 2 mm.

Eine vereinfachte, schematische Darstellung des Ausatemsystems 3 in einer beispielhaften Ausführungsform ist in Figur 9 gezeigt. Das Ausatemsystem 3 besteht im Wesentlichen aus einer ersten Wand 301 und einer zweiten Wand 302, welche nebeneinander angeordnet sind, wobei in zumindest einer der Wände 301, 302 Rillen 304 angeordnet sind, welche zusammen mit der jeweils anderen Wand 301, 302 Kanäle 305 ausbilden. Beispielhaft sind in der dargestellten Ausführungsform die Rillen 304 in der Innenfläche 301a der ersten Wand 301 versenkt angeordnet. Entsprechend bilden die Rillen 304 zusammen mit der Außenfläche 302b der zweiten Wand 302 die Kanäle 305 aus. In der gezeigten Ausführungsform ist beispielhaft kein Kragen 306 an der Außenfläche 301b der ersten Wand 301 angeordnet. In manchen Ausführungsformen, bei denen kein Kragen 306 an der ersten Wand 301 angeordnet ist, ist eine Stufe 321 zwischen der oberen Fläche 301c der ersten Wand und der Oberfläche 204 des Maskenkörpers 2 ausgebildet bzw. angeordnet.

Die zweite Wand 302 ist mit der Außenfläche 302b an Innenfläche 301a der ersten Wand 301 angeordnet. An den Stellen, an denen die Innenfläche 301a durch die Rillen 304 unterbrochen ist, sind so die Kanäle 305 ausgebildet. Beispielhaft ist an der Außenfläche 302b der zweiten Wand 302 ein Kragen 307 angeordnet. Die Unterseite 307b liegt beispielhaft auf der oberen Fläche 301c auf. An der Unterseite 307b des Kragens 307 sind stellenweise Aussparungen 308 angeordnet bzw. ausgebildet, wobei die Aussparungen 308 von der Außenseite 307c beziehungsweise der Kante zwischen Außenseite 307c und Unterseite 307b bis zur Außenfläche 302b bzw. der Kante zwischen Außenfläche 302b und der Unterseite 307b reichen.

In den Bereichen, in denen die Aussparungen 308 ausgebildet sind, bilden die Aussparungen 308 zusammen mit der oberen Fläche 301c Spalte 309 oberhalb der oberen Fläche 301c. Bevorzugt sind die Aussparungen 308 so angeordnet, dass diese in den Bereichen angeordnet sind, in denen auch die Rillenanordnungen 303 beziehungsweise die Rillen 304 angeordnet sind. Über die Spalte 309 wird beispielsweise das Atemgas, welche durch die Kanäle 305 aus dem Innenraum 101 der Patientenschnittstelle 1 strömt, in den Außenbereich 102 geleitet. Letztlich bilden also die Spalte 309 zusammen mit den Kanälen 305 die gasleitende Verbindung zwischen dem Innenraum 101 und dem Außenbereich 102. In manchen Ausführungsformen ist zu jeder Rille 304 eine Aussparung 308 ausgebildet. In manchen Ausführungsformen ist zu jeder Rillenanordnung 303 eine Aussparung 308 ausgebildet, wobei sich die Aussparung 308 beziehungsweise der Spalt 309 über die gesamte Breite der Rillenanordnung 303, also mindestens von den äußeren Kanten der äußeren Rillen 304, erstreckt. In manchen Ausführungsformen kann sich die Aussparung 308 auch mit bis zu 10% der Gesamtbreite der Rillenanordnung 303 darüber hinaus erstrecken. In manchen Ausführungsformen ist für jedes Paar von Rillenanordnungen 304 eine Aussparung 308 ausgebildet, wobei sich die Aussparung 308 beziehungsweise der Spalt 309 über die gesamte Breite des Paares, also mindestens von der äußeren Kante der einen Rillenanordnung 303 zur äußeren Kante der anderen Rillenanordnung 303, erstreckt. In manchen Ausführungsformen kann sich die Aussparung 308 auch mit bis zu 10% der Gesamtbreite des Paares von Rillenanordnungen 303 darüber hinaus erstrecken.

Die Unterseite 307 steht in einem Winkel B zu der Außenfläche 302b der zweiten Wand 302, wobei der Winkel B dem Winkel A entspricht, in welchem die obere Fläche 301c zu der Innenfläche 301a steht. Die Außenfläche 302b der zweiten Wand verläuft parallel zu der Innenfläche 301a der ersten Wand. Die obere Fläche 301c und die Unterseite 307b verlaufen im Wesentlichen parallel zu einander.

Die Aussparungen 308 weisen beispielsweise eine Tiefe in einem Bereich von 0,1 mm bis 3 mm auf. Entsprechend sind die Aussparungen 308 also so ausgebildet, dass ein Spalt 309 mit einer Höhe H von 0,1 mm bis 3 mm entsteht.

In manchen Ausführungsformen sind die Rillen 304 beziehungsweise die Rillenanordnungen 303 beispielsweise alternativ oder ergänzend in der Außenfläche 302b der zweiten Wand 302 versenkt angeordnet. In manchen Ausführungsformen sind die Aussparungen 308 alternativ oder ergänzend in der oberen Fläche 301c angeordnet. Sind die Rillen 304 beziehungsweise die Rillenanordnungen 303 in der Außenfläche 302b der zweiten Wand 302 versenkt angeordnet, so sind die Ausführungen, welche sich auf eine Anordnung der Rillenanordnungen 303 in der ersten Wand 301 beziehen, im gleichen Maße anzuwenden.

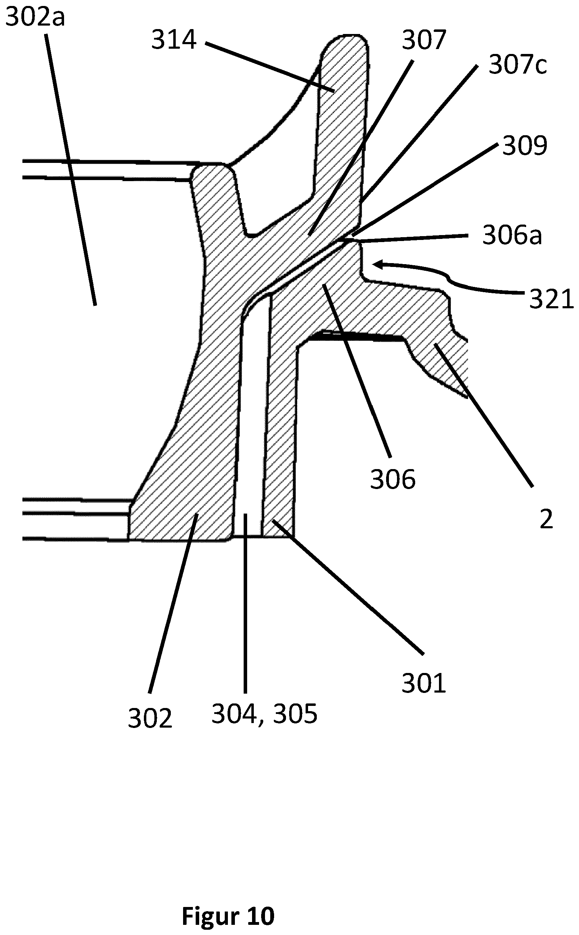

Eine beispielhafte Ausführungsform der ersten Wand 301 und der zweiten Wand 302 ist in einem Schnitt in Figur 10 dargestellt. Der Schnitt folgt dabei im Wesentlichen der Schnittkante X-X aus Figur 5, wobei zusätzlich zum Maskenkörper 2 mit der ersten Wand 301 auch der Verschluss 7 mit der zweiten Wand 302 dargestellt ist.

Im Unterschied zu der in Figur 9 gezeigten Ausführungsform ist an der Außenfläche 301b der ersten Wand 301 der Kragen 306 angeordnet. Zwischen dem Kragen 306 und dem Maskenkörper 2 ist beispielhaft eine Stufe 321 angeordnet. Die Stufe 321 verläuft in manchen Ausführungsformen entlang des gesamten Kragens 306. In manchen Ausführungsformen verläuft die Stufe 321 nicht entlang des gesamten Kragens 306, zumindest aber in den Bereichen, in denen auch Rillenanordnungen 303 und/oder Aussparungen 308 angeordnet sind. Beispielsweise ist die im Nasenbereich 323 nur teilweise ausgebildet.

Beispielhaft sind in der ersten Wand 301 Rillen 304 angeordnet, welche zusammen mit der zweiten Wand 302 die Kanäle 305 bilden. Durch die Aussparungen 308 in der Unterseite 307b des Kragens 307 bilden sich Spalte 309. Durch die Kanäle 305 und Spalte 309 kann Atemgas aus dem Innenraum 101 der Patientenschnittstelle 1 in den Außenbereich 102 entweichen. Auf der Oberseite 307a des Kragens 307 sind abschnittsweise Griffwände 314 angeordnet, welche beispielhaft parallel zu der Außenfläche 302b der zweiten Wand 302 stehen.

Die Innenfläche 302a der zweiten Wand 302 stellt beispielhaft eine (hohle) Teilkugel dar, wobei die Teilkugel zumindest einen Äquator C aufweist. Die Innenfläche 302a ist so ausgebildet und eingerichtet, dass ein Gelenkkopf 601 aufgenommen werden kann. Beispielhaft stellt die Innenfläche 302a dadurch das Gelenklager 602 eines Kugelgelenks dar. In manchen Ausführungsformen ist die Innenfläche 302a nicht kugelförmig, sondern beispielsweise gerade, optional mit Befestigungselementen zum Fixieren eines Rohrs. Der Krümmungsradius der Außenfläche 604b der dritten Wand 604 beträgt beispielhaft 15,5 mm (+/- 5%), kann aber allgemein zwischen 10 mm und 20 mm, bevorzugt zwischen 12,5 mm und 17,5 mm liegen. Entsprechend liegt der Krümmungsradius der Innenfläche 302a beispielhaft ebenfalls bei etwa 15,5 mm (+/- 5%), im Allgemeinen aber passend zum Krümmungsradius der Außenfläche 604b zwischen 10 mm und 20 mm, bevorzugt zwischen 12,5 mm und 17,5 mm.

Figuren 11 und 12 zeigen eine beispielhafte Ausführungsform des Verschlusses 7 mit der zweiten Wand 302.

Eine Ansicht mit Blick auf die Unterseite 307b des Kragens 307 ist in Figur 11 dargestellt. Die zweite Wand 302 umgibt eine Öffnung 318, in welche beispielsweise ein Gelenkkopf 601 eingesteckt werden kann. In die Außenfläche 302b und die Unterseite 302c der zweiten Wand 302 sind Verschlussschlitze 317 angeordnet. Die Verschlussschlitze 317 sind beispielhaft so angeordnet, dass sie der Anordnung der Verschlusszapfen 311 an der Innenfläche 301a der ersten Wand entsprechen. So kann der Verschluss 7 in nur einer Position beziehungsweise Orientierung in die Öffnung 320 eingesetzt werden.

In der Unterseite 307b des Kragens 307, welche an der Außenfläche 302b der zweiten Wand 302 angeordnet ist, sind Aussparungen 308 angeordnet. Beispielhaft sind drei Aussparungen 308 angeordnet, welche von der Position den (Paaren der) Rillenanordnungen 303 in der ersten Wand 301 entsprechen. In manchen Ausführungsformen ist an der Unterseite 307b des Kragens 307 eine große Aussparung 308 angeordnet, welche über alle Rillenanordnungen 303 reicht. In manchen Ausführungsformen des Verschlusses 7 ist insbesondere im Bereich des Vorsprungs 315 keine Aussparung 308 in der Unterseite 307b angeordnet.

An der Außenseite 307c des Kragens ist beispielhaft ein Vorsprung 315 angeordnet. An diesem Vorsprung ist ein Steg 319 angeordnet, an dessen Unterseite eine Nase 316 ausgebildet ist. Diese Nase 316 kann hinter der Erhebung 313 in der Vertiefung 312 einrasten und verhindert so ein unbeabsichtigtes Drehen des Verschlusses 7 in der Öffnung 320.

Figur 12 zeigt eine Seitansicht einer beispielhaften Ausführungsform des Verschlusses 7 mit Blick auf den Vorsprung 315. In der Darstellung sind seitlich die Aussparungen 308 in der Unterseite 307b des Kragens 307 zu erkennen. Im Bereich des Vorsprungs 315 ist in der Unterseite 307b keine Aussparung angedacht. An dem Vorsprung 315 ist ein Steg 319 mit einer Nase 316 angeordnet. Die Nase 316 ist dabei beispielhaft nicht in der Mitte des Stegs 319, sondern leicht versetzt angeordnet, damit sie hinter der mittig in der Vertiefung 312 angeordneten Erhebung 313 einrasten kann.

In der Außenfläche 302b der zweiten Wand 302 sind Verschlussschlitze 317 angeordnet, welche zusammen mit den Verschlusszapfen 311 an der Innenfläche 301a der ersten Wand 301 einen Bajonett-Verschluss ergeben.

Die Außenfläche 302b der zweiten Wand 302 verläuft leicht konisch, wobei die Außenfläche 302b parallel zu der Innenfläche 301a der ersten Wand 301 verläuft. Dadurch lässt sich ein einfacheres Einsetzen des Verschlusses 7 in die von der ersten Wand 301 umgebenen Öffnung 320 erreichen.

An den auf dem Kragen 307 angeordneten Griffwänden 314 sind beispielhaft Griffstrukturen 325 angeordnet. Beispielsweise sind die Griffstrukturen 325 sind beispielsweise als längliche Erhebungen auf der Griffwand 314 ausgebildet.

### Bezugszeichenliste

- 1: Patientenschnittstelle
- 2: Maskenkörper
- 3: Ausatemeinrichtung
- 4: Maskenpolster
- 5: Schlauchanschluss
- 6: Kugelgelenk
- 7: Verschluss
- 101: Innenraum
- 102: Außenbereich
- 201: Nasenbereich
- 202: Basisbereich
- 203: Seitenbereich
- 204: Oberfläche
- 301: Wand
- 301a: Innenfläche
- 301b: Außenfläche
- 301c: obere Fläche
- 301d: untere Fläche
- 302: Wand
- 302a: Innenfläche
- 302b: Außenfläche
- 303: Rillenanordnung
- 304: Rille
- 305: Kanal
- 306: Kragen
- 306a: Außenkante
- 307: Kragen
- 307a: Oberseite
- 307b: Unterseite
- 307c: Außenseite
- 308: Aussparung
- 309: Spalt
- 310: Einkerbung
- 311: Verschlusszapfen
- 312: Vertiefung
- 313: Erhebung
- 314: Griffwand
- 315: Vorsprung
- 316: Nase
- 317: Verschlussschlitz
- 318: Öffnung
- 319: Steg
- 320: Öffnung
- 321: Stufe
- 322: Seitenbereich
- 323: Nasenbereich
- 324: Basisbereich
- 325: Griffstruktur
- 601: Gelenkkopf
- 602: Gelenklager
- 603: Rohrstück
- 604: Wand
- 604a: Innenfläche
- 604b: Außenfläche
- A: Winkel
- B: Winkel
- C: Äquator
- H: Höhe
- S1: Strömungsweg
- S1a: Verlängerung
- S2: Strömungsweg
- S2a: Abzweig
- S3: Teilströmungsweg
- S3a: Verlängerung

## Patentansprüche

1. Ausatemsystem (3) für eine Patientenschnittstelle (1), umfassend zumindest zwei Wände (301, 302), wobei die Wände (301, 302) zumindest abschnittsweise nebeneinander angeordnet sind, wobei das Ausatemsystem zumindest zwei Strömungswege (S1, S2) zur Strömung von Atemgas aus einem Innenraum (101) der Patientenschnittstelle (1) aufweist, wobei der erste Strömungsweg (S1) zumindest teilweise zwischen der ersten Wand (301) und der zweiten Wand (302) verläuft und der erste Strömungsweg (S1) zum zumindest zeitweisen Abbau von Atemgasdruck ausgebildet ist und wobei der zweite Strömungsweg (S2) zumindest teilweise von der zweiten Wand (302) umschlossen wird und der zweite Strömungsweg (S2) zumindest teilweise zur zumindest zeitweisen Strömung von Atemgas in den Innenraum (101) der Patientenschnittstelle (1) ausgebildet ist, **dadurch gekennzeichnet, dass** in zumindest einer der Wände (301, 302) zumindest vier Rillenanordnungen (303) angeordnet sind, wobei jede Rillenanordnung (303) zumindest zwei Rillen (304) umfasst und wobei die Rillen (304) zusammen mit der anderen Wand (301, 302) Kanäle (305) ausbilden, wobei die Rillenanordnungen (303) immer paarweise angeordnet sind und der Abstand zwischen zwei Paaren von Rillenanordnungen (303) größer ist als der Abstand der zwei Rillenanordnungen eines jeweiligen Paars untereinander.

2. Ausatemsystem (3) nach Anspruch 1, **dadurch gekennzeichnet, dass** das Ausatemsystem (3) eine dritte Wand (604) umfasst, wobei die dritte Wand (604) den zweiten Strömungsweg (S2) zumindest teilweise umschließt.

3. Ausatemsystem (3) nach Anspruch 2, **dadurch gekennzeichnet, dass** der zweite Strömungsweg (S2) zumindest zeitweise einen Abzweig (S2a) aufweist, wobei der Abzweig (S2a) zwischen der der zweiten Wand (302) und der dritten Wand (604) zumindest abschnittsweise koaxial zu dem zweiten Strömungsweg (S2) verläuft und zur zumindest zeitweisen Strömung von Atemgas aus dem Innenraum (101) der Patientenschnittstelle (1) und zum zumindest zeitweisen Abbau von Atemgasdruck ausgebildet ist.

4. Ausatemsystem (3) nach Anspruch 3, **dadurch gekennzeichnet, dass** der erste Strömungsweg (S1), der zweite Strömungsweg (S2) und der Abzweig (S2a) zumindest teilweise koaxial zueinander verlaufen, wobei der zweite Strömungsweg (S2) zentral verläuft und der Abzweig (S2a) zumindest teilweise um den zweiten Strömungsweg (S2) verläuft und wobei der erste Strömungsweg (S1) zumindest teilweise um den zweiten Strömungsweg (S2) und den Abzweig (S2a) verläuft und wobei der erste Strömungsweg (S1) zumindest einen Teilströmungsweg (S3) aufweist, welcher im Wesentlichen parallel zum ersten Strömungsweg (S1) verläuft.

5. Ausatemsystem (3) nach zumindest einem der vorhergehenden Ansprüche, **dadurch gekennzeichnet, dass** die zumindest eine Rillenanordnung (303) in einer Innenfläche (301a) der ersten Wand (301) angeordnet ist und die Rillen (304) zusammen mit einer Außenfläche (302b) der zweiten Wand (302) die Kanäle (305) bilden oder zumindest eine Rillenanordnung (303) in der Außenfläche (302b) der zweiten Wand (301) angeordnet ist und die Rillen (304) zusammen mit Innenfläche (301a) der ersten Wand (301) die Kanäle (305) bilden.

6. Ausatemsystem (3) nach Anspruch 4, **dadurch gekennzeichnet, dass** durch die Kanäle (305) der zumindest eine Teilströmungsweg (S3) des ersten Strömungsweges (S1) verläuft und die Kanäle (305) den Innenraum (101) der Patientenschnittstelle (1) mit dem Außenbereich (102) der Patientenschnittstelle (1) gasleitend verbinden.

7. Ausatemsystem (3) nach zumindest einem der vorhergehenden Ansprüche, **dadurch gekennzeichnet, dass** an der Außenfläche (302b) der zweiten Wand (302) ein Kragen (307) angeordnet ist, wobei der Kragen (307) mit einer Unterseite (307b) formschlüssig auf einer oberen Fläche (301c) der ersten Wand (301) angeordnet ist, und wobei die Unterseite (307b) des Kragens (307) und/oder die obere Fläche (301c) der ersten Wand zumindest eine Aussparung (308) aufweist, welche zusammen mit der oberen Fläche (301c) der ersten Wand (301) und/oder der Unterseite (307b) des Kragens (307) zumindest einen Spalt (309) bildet.

8. Ausatemsystem (3) nach Anspruch 7 rückbezogen auf Anspruch 4, **dadurch gekennzeichnet, dass** an der Unterseite (307b) des Kragens (307) eine Verlängerung (S1a) des ersten Strömungsweges (S1) verläuft und in dem zumindest einen Spalt (309) zumindest teilweise eine Verlängerung (S3a) des zumindest einen Teilströmungswegs (S3) verläuft und der Strömungsquerschnitt des zumindest einen Teilströmungswegs (S3) nicht gleich dem Querschnitt der Verlängerung (S3a) ist.

9. Ausatemsystem (3) nach Anspruch 7, **dadurch gekennzeichnet, dass** sich die zumindest eine Aussparung (308) an der Unterseite (307b) des Kragens (307) von einer Außenfläche (307c) des Kragens (307) bis zur Außenfläche (302b) der zweiten Wand (302) erstreckt.

10. Ausatemsystem (3) nach zumindest einem der vorhergehenden Ansprüche, **dadurch gekennzeichnet, dass** an der Außenfläche (301b) der ersten Wand (301) ein Kragen (306) angeordnet ist, wobei der Kragen (306) zusammen mit der Wand (301) eine gemeinsame obere Fläche (301c) aufweist, wobei zumindest eine Aussparung (308) in der oberen Fläche (301c) der ersten Wand (301) und des Kragens (306) ausgebildet ist, wobei sich die Aussparung von einer Außenkante (306a) des Kragens (306) bis zur Innenfläche (301a) der ersten Wand (301) erstreckt.

11. Ausatemsystem (3) nach zumindest einem der vorhergehenden Ansprüche, **dadurch gekennzeichnet, dass** zumindest eine Aussparung (308) und/oder Spalt (309) oberhalb zumindest einer Rillenanordnung (303) angeordnet ist und der Spalt (309) gasleitend mit den Rillen (304) verbunden ist.

12. Ausatemsystem (3) nach zumindest einem der vorhergehenden Ansprüche, **dadurch gekennzeichnet, dass** eine Rillenanordnung (303) höchstens 8 Rillen umfasst, wobei die Zahl der Rillen (304) je Rillenanordnung (303) unabhängig voneinander ist

13. Ausatemsystem (3) nach zumindest einem der vorhergehenden Ansprüche, **dadurch gekennzeichnet, dass** die zweite Wand (302) im Wesentlichen rohrförmig ausgebildet ist, wobei die Innenfläche (302a) der zweiten Wand (302) einen kreisförmigen Querschnitt aufweist und die erste Wand (301) im Wesentlichen rohrförmig ausgebildet ist und die Innenfläche (302a) der zweiten Wand (302) einen Kugelausschnitt darstellt.

14. Ausatemsystem (3) nach zumindest einem der vorhergehenden Ansprüche, **dadurch gekennzeichnet, dass** die Innenfläche (301a) der ersten Wand (301) und die Außenfläche (302b) der zweiten Wand (302) parallel zueinander verlaufen und konisch zusammenlaufen.

15. Ausatemsystem (3) nach Anspruch 10 rückbezogen auf Anspruch 7, **dadurch gekennzeichnet, dass** die erste Wand (301) zusammen mit dem Kragen (306) eine Trichterform bildet, wobei die obere Fläche (301c) in einem Winkel A zu der Innenfläche (301a) steht, wobei der Winkel A zwischen 0° und 90° liegt, bevorzugt in einem Bereich von 22° bis 75° und die zweite Wand (302) zusammen mit dem Kragen (307) eine Trichterform bildet, wobei die Unterseite (307b) des Kragens (307) in einem Winkel B zu der Außenfläche (302b) steht, wobei der Winkel B zwischen 0° und 90° liegt, bevorzugt in einem Bereich von 22° bis 75°, wobei der Winkel A gleich dem Winkel B ist.

16. Ausatemsystem (3) nach zumindest einem der vorhergehenden Ansprüche, **dadurch gekennzeichnet, dass** die Rillen (304) einen im Wesentlichen rechteckigen Querschnitt aufweisen, optional mit abgerundeten Ecken und/oder einem aufgesetzten Teilkreis, wobei die Rillen (304) eine maximale Breite von 1,25 mm und eine maximal Tiefe von 1,25 mm aufweisen.

17. Ausatemsystem (3) nach zumindest einem der vorhergehenden Ansprüche, **dadurch gekennzeichnet, dass** an der Innenfläche (301a) der ersten Wand (301) zumindest drei Verschlusszapfen (311) angeordnet sind und in der Außenfläche (302b) der zweiten Wand (302) eine entsprechende Anzahl an Verschlussschlitzen (317) angeordnet sind, wobei die Verschlusszapfen (311) der ersten Wand (301) unregelmäßig angeordnet und die Verschlussschlitze (317) in der zweiten Wand (302) im gleichen Maße unregelmäßig angeordnet sind, sodass die Position der Verschlussschlitze (317) und der Verschlusszapfen (311) in nur einer Stellung der ersten Wand (301) zur zweiten Wand (302) übereinstimmen.

18. Patientenschnittstelle (1), **dadurch gekennzeichnet, dass** die Patientenschnittstelle (1) ein Ausatemsystem (3) nach zumindest einem der vorhergehenden Ansprüche und ein Kugelgelenk (6) umfasst.

19. Patientenschnittstelle (1) nach Anspruch 18, wobei das Kugelgelenk (6) zumindest einen Gelenkkopf (601) und ein Gelenklager (602) umfasst, **dadurch gekennzeichnet, dass** der Gelenkkopf (601) im Wesentlichen durch die dritte Wand (604) dargestellt wird und das Gelenklager (602) im Wesentlichen durch die Innenfläche (302a) der zweiten Wand (302) gebildet wird.

20. Patientenschnittstelle (1) nach Anspruch 19, **dadurch gekennzeichnet, dass** die Innenfläche (302a) der zweiten Wand (302) kugelförmig ausgebildet ist und die Außenfläche (604b) der dritten Wand (604) dazu komplementär kugelförmig ausgebildet ist, wobei die Krümmungsradien der Außenfläche (604b) und der Innenfläche (302a) im Wesentlichen gleich sind und/oder geringfügig (<1%) voneinander abweichen und die Krümmungsradien der Innenfläche (302a) der zweiten Wand (302) und der Außenfläche (604b) der dritten Wand (604) so gewählt sind, dass eine Bewegung der dritten Wand (604) relativ zur zweiten Wand (302) möglich ist.

## Claims

1. An exhalation system (3) for a patient interface (1), comprising at least two walls (301, 302), wherein the walls (301, 302) are arranged next to each other at least in portions, wherein the exhalation system has at least two flow paths (S1, S2) for the flow of respiratory gas from an interior (101) of the patient interface (1), wherein the first flow path (S1) runs at least partially between the first wall (301) and the second wall (302), and the first flow path (S1) is formed to at least temporarily reduce respiratory gas pressure, and wherein the second flow path (S2) is at least partially surrounded by the second wall (302), and the second flow path (S2) is formed at least partially for the at least temporary flow of respiratory gas into the interior (101) of the patient interface (1), **characterized in that** at least four groove arrangements (303) are arranged in at least one of the walls (301, 302), wherein each groove arrangement (303) comprises at least two grooves (304) and wherein the grooves (304), together with the other wall (301, 302) form channels (305), wherein the groove arrangements (303) are always arranged in pairs and the distance between two pairs of groove arrangements (303) is greater than the distance of the two groove arrangements of each pair from one another.

2. The exhalation system (3) according to claim 1, **characterized in that** the exhalation system (3) comprises a third wall (604), wherein the third wall (604) at least partially surrounds the second flow path (S2).

3. The exhalation system (3) according to claim 2, **characterized in that** the second flow path (S2) has a branch (S2a) at least temporarily, wherein the branch (S2a) runs between the second wall (302) and the third wall (604) at least in portions coaxially to the second flow path (S2) and is formed for at least temporary flow of respiratory gas from the interior (101) of the patient interface (1) and to at least temporarily reduce respiratory gas pressure.

4. The exhalation system (3) according to claim 3, **characterized in that** the first flow path (S1), the second flow path (S2), and the branch (S2a) run at least partially coaxially to each other, wherein the second flow path (S2) runs centrally and the branch (S2a) runs at least partially around the second flow path (S2), and wherein the first flow path (S1) runs at least partially around the second flow path (S2) and the branch (S2a), and wherein the first flow path (S1) has at least one partial flow path (S3), which runs substantially parallel to the first flow path (S1).

5. The exhalation system (3) according to at least one of the preceding claims, **characterized in that** the at least one groove arrangement (303) is arranged in an inner face (301a) of the first wall (301) and the grooves (304), together with an outer face (302b) of the second wall (302), form the channels (305), or at least one groove arrangement (303) is arranged in the outer face (302b) of the second wall (301) and the grooves (304), together with an inner face (301a) of the first wall (301), form the channels (305).

6. The exhalation system (3) according to claim 4, **characterized in that** the at least one partial flow path (S3) of the first flow path (S1) runs through the channels (305) and the channels (305) connect the interior (101) of the patient interface (1) to the outer region (102) of the patient interface (1) in a gas-conducting manner.

7. The exhalation system (3) according to at least one of the preceding claims, **characterized in that** a collar (307) is arranged on the outer face (302b) of the second wall (302), wherein an underside (307b) of the collar (307) is arranged in a positivelocking manner on an upper face (301c) of the first wall (301), and wherein the underside (307b) of the collar (307) and/or the upper face (301c) of the first wall has at least one recess (308), which forms at least one gap (309) together with the upper face (301c) of the first wall (301) and/or the underside (307b) of the collar (307).

8. The exhalation system (3) according to claim 7 with reference to claim 4, **characterized in that** an extension (S1a) of the first flow path (S1) runs on the underside (307b) of the collar (307) and an extension (S3a) of the at least one partial flow path (S3) runs at least partially in the at least one gap (309), and the flow cross-section of the at least one partial flow path (S3) is not equal to the cross-section of the extension (S3a).

9. The exhalation system (3) according to claim 7, **characterized in that** the at least one recess (308) extends on the underside (307b) of the collar (307) from an outer face (307c) of the collar (307) to the outer face (302b) of the second wall (302).

10. The exhalation system (3) according to at least one of the preceding claims, **characterized in that** a collar (306) is arranged on the outer face (301b) of the first wall (301), wherein the collar (306) has a common upper face (301c) with the wall (301), wherein at least one recess (308) is formed in the upper face (301c) of the first wall (301) and of the collar (306), wherein the recess extends from an outer edge (306a) of the collar (306) to the inner face (301a) of the first wall (301).

11. The exhalation system (3) according to at least one of the preceding claims, **characterized in that** at least one recess (308) and/or gap (309) is arranged above at least one groove arrangement (303) and the gap (309) is connected to the grooves (304) in a gas-conducting manner.

12. The exhalation system (3) according to at least one of the preceding claims, **characterized in that** a groove arrangement (303) comprises at most 8 grooves, wherein the number of grooves (304) per groove arrangement (303) is independent of each other.

13. The exhalation system (3) according to at least one of the preceding claims, **characterized in that** the second wall (302) is substantially tubular, wherein the inner face (302a) of the second wall (302) has a circular cross-section and the first wall (301) is substantially tubular and the inner face (302a) of the second wall (302) constitutes a spherical cutout.

14. The exhalation system (3) according to at least one of the preceding claims, **characterized in that** the inner face (301a) of the first wall (301) and the outer face (302b) of the second wall (302) run parallel to each other and come together conically.

15. The exhalation system (3) according to claim 10 with reference to claim 7, **characterized in that** the first wall (301), together with the collar (306), forms a funnel shape, wherein the upper face (301c) is at an angle A to the inner face (301a), wherein the angle A is between 0° and 90°, preferably in a range from 22° to 75°, and the second wall (302), together with the collar (307) forms a funnel shape, wherein the underside (307b) of the collar (307) is at an angle B to the outer face (302b), wherein the angle B is between 0° and 90°, preferably in a range from 22° to 75°, wherein the angle A is equal to the angle B.

16. The exhalation system (3) according to at least one of the preceding claims, **characterized in that** the grooves (304) have a substantially rectangular cross-section, optionally with rounded corners and/or a placed-on partial circle, wherein the grooves (304) have a maximum width of 1.25 mm and a maximum depth of 1.25 mm.

17. The exhalation system (3) according to at least one of the preceding claims, **characterized in that** at least three closure pins (311) are arranged on the inner face (301a) of the first wall (301) and a corresponding number of closure slots (317) are arranged in the outer face (302b) of the second wall (302), wherein the closure pins (311) of the first wall (301) are arranged irregularly and the closure slots (317) in the second wall (302) are arranged irregularly in equal measure, such that the position of the closure slots (317) and of the closure pins (311) match in only one position of the first wall (301) relative to the second wall (302).

18. A patient interface (1), **characterized in that** the patient interface (1) comprises an exhalation system (3) according to at least one of the preceding claims and a ball joint (6).

19. The patient interface (1) according to claim 18, wherein the ball joint (6) comprises at least one joint head (601) and one joint bearing (602), **characterized in that** the joint head (601) is constituted substantially by the third wall (604) and the joint bearing (602) is formed substantially by the inner face (302a) of the second wall (302).

20. The patient interface (1) according to claim 19, **characterized in that** the inner face (302a) of the second wall (302) is spherical and the outer face (604b) of the third wall (604) is spherical in a manner that is complementary thereto, wherein the radii of curvature of the outer face (604b) and the inner face (302a) are substantially equal and/or deviate slightly (<1%) from each other, and the radii of curvature of the inner face (302a) of the second wall (302) and the outer face (604b) of the third wall (604) are selected such that a movement of the third wall (604) relative to the second wall (302) is possible.

## Revendications

1. Système d'exhalation (3) pour une interface patient (1), comportant au moins deux parois (301, 302), dans lequel les parois (301, 302) sont disposées côte à côte au moins par sections, dans lequel le système d'exhalation présente au moins deux trajets d'écoulement (S1, S2) pour l'écoulement de gaz respiratoire hors d'un espace intérieur (101) de l'interface patient (1), dans lequel le premier trajet d'écoulement (S1) s'étend au moins partiellement entre la première paroi (301) et la deuxième paroi (302) et le premier trajet d'écoulement (S1) est conçu pour la réduction au moins temporaire de la pression de gaz respiratoire et dans lequel le deuxième trajet d'écoulement (S2) est entouré au moins partiellement par la deuxième paroi (302) et le deuxième trajet d'écoulement (S2) est conçu au moins partiellement pour l'écoulement au moins temporaire de gaz respiratoire dans l'espace intérieur (101) de l'interface patient (1), **caractérisé en ce qu'**au moins quatre ensembles de rainures (303) sont disposés dans l'une au moins des parois (301, 302), dans lequel chaque ensemble de rainures (303) comporte au moins deux rainures (304) et dans lequel les rainures (304) forment des canaux (305) conjointement avec l'autre paroi (301, 302), dans lequel les ensembles de rainures (303) sont toujours disposés par paires et la distance entre deux paires d'ensembles de rainures (303) est plus grande que la distance entre les deux ensembles de rainures d'une paire respective.

2. Système d'exhalation (3) selon la revendication 1, **caractérisé en ce que** le système d'exhalation (3) comporte une troisième paroi (604), dans lequel la troisième paroi (604) entoure au moins partiellement le deuxième trajet d'écoulement (S2).

3. Système d'exhalation (3) selon la revendication 2, **caractérisé en ce que** le deuxième trajet d'écoulement (S2) présente au moins temporairement une ramification (S2a), dans lequel la ramification (S2a) s'étend au moins par sections coaxialement au deuxième trajet d'écoulement (S2) entre la deuxième paroi (302) et la troisième paroi (604) et est conçu pour l'écoulement au moins temporaire de gaz respiratoire hors de l'espace intérieur (101) de l'interface patient (1) et pour la réduction au moins temporaire de la pression de gaz respiratoire.

4. Système d'exhalation (3) selon la revendication 3, **caractérisé en ce que** le premier trajet d'écoulement (S1), le deuxième trajet d'écoulement (S2) et la ramification (S2a) s'étendent au moins partiellement coaxialement les uns par rapport aux autres, dans lequel le deuxième trajet d'écoulement (S2) s'étend de façon centrale et la ramification (S2a) s'étend au moins partiellement autour du deuxième trajet d'écoulement (S2) et dans lequel le premier trajet d'écoulement (S1) s'étend au moins partiellement autour du deuxième trajet d'écoulement (S2) et de la ramification (S2a) et dans lequel le premier trajet d'écoulement (S1) présente au moins un trajet d'écoulement partiel (S3), lequel s'étend essentiellement parallèlement au premier trajet d'écoulement (S1).

5. Système d'exhalation (3) selon l'une au moins des revendications précédentes, **caractérisé en ce que** l'au moins un ensemble de rainures (303) est disposé dans une surface intérieure (301a) de la première paroi (301) et les rainures (304) forment les canaux (305) conjointement avec une surface extérieure (302b) de la deuxième paroi (302) ou au moins un ensemble de rainures (303) est disposé dans la surface extérieure (302b) de la deuxième paroi (301) et les rainures (304) forment les canaux (305) conjointement avec la surface intérieure (301a) de la première paroi (301).

6. Système d'exhalation (3) selon la revendication 4, **caractérisé en ce que** l'au moins un trajet d'écoulement partiel (S3) du premier trajet d'écoulement (S1) s'étend à travers les canaux (305) et les canaux (305) relient l'espace intérieur (101) de l'interface patient (1) à la région extérieure (102) de l'interface patient (1) de façon à guider du gaz.

7. Système d'exhalation (3) selon l'une au moins des revendications précédentes, **caractérisé en ce qu'**un col (307) est disposé sur la surface extérieure (302b) de la deuxième paroi (302), dans lequel le col (307) est disposé avec une côté inférieur (307b) sur une surface supérieure (301c) de la première paroi (301) par complémentarité de forme, et dans lequel le côté inférieur (307b) du col (307) et/ou la surface supérieure (301c) de la première paroi présentent un évidement (308), lequel forme au moins une fente (309) conjointement avec la surface supérieure (301c) de la première paroi (301) et/ou le côté inférieur (307b) du col (307).

8. Système d'exhalation (3) selon la revendication 7 lorsqu'elle dépend de la revendication 4, **caractérisé en ce qu'**un prolongement (S1a) du premier trajet d'écoulement (S1) s'étend sur le côté inférieur (307b) du col (307) et un prolongement (S3a) de l'au moins un trajet d'écoulement partiel (S3) s'étend au moins partiellement dans l'au moins une fente (309) et la section transversale d'écoulement de l'au moins un trajet d'écoulement partiel (S3) est différente de la section transversale du prolongement (S3a).

9. Système d'exhalation (3) selon la revendication 7, **caractérisé en ce que** l'au moins un évidement (308) se développe à partir d'une surface extérieure (307c) du col (307) jusqu'à la surface extérieure (302b) de la deuxième paroi (302) sur le côté inférieur (307b) du col (307).

10. Système d'exhalation (3) selon l'une au moins des revendications précédentes, **caractérisé en ce qu'**un col (306) est disposé sur la surface extérieure (301b) de la première paroi (301), dans lequel le col (306) présente une surface supérieure (301c) commune conjointement avec la paroi (301), dans lequel au moins un évidement (308) est formé dans la surface supérieure (301c) de la première paroi (301) et du col (306), dans lequel l'évidement se développe à partir d'un bord extérieur (306a) du col (306) jusqu'à la surface intérieure (301a) de la première paroi (301).

11. Système d'exhalation (3) selon l'une au moins des revendications précédentes, **caractérisé en ce qu'**au moins un évidement (308) et/ou une fente (309) sont disposés au-dessus d'au moins un ensemble de rainures (303) et la fente (309) est reliée aux rainures (304) de manière à guider du gaz.

12. Système d'exhalation (3) selon l'une au moins des revendications précédentes, **caractérisé en ce qu'**un ensemble de rainures (303) comporte au maximum 8 rainures, dans lequel le nombre des rainures (304) par ensemble de rainures (303) est indépendant d'un ensemble à l'autre.

13. Système d'exhalation (3) selon l'une au moins des revendications précédentes, **caractérisé en ce que** la deuxième paroi (302) est conçue essentiellement en forme de tuyau, dans lequel la surface intérieure (302a) de la deuxième paroi (302) présente une section transversale circulaire et la première paroi (301) est conçue essentiellement en forme de tuyau et la surface intérieure (302a) de la deuxième paroi (302) représente une découpe sphérique.

14. Système d'exhalation (3) selon l'une au moins des revendications précédentes, **caractérisé en ce que** la surface intérieure (301a) de la première paroi (301) et la surface extérieure (302b) de la deuxième paroi (302) s'étendent parallèlement l'une à l'autre et convergent de façon conique.

15. Système d'exhalation (3) selon la revendication 10 lorsqu'elle dépend de la revendication 7, **caractérisé en ce que** la première paroi (301) constitue une forme d'entonnoir conjointement avec le col (306), dans lequel la surface supérieure (301c) se trouve à un angle A par rapport à la surface intérieure (301a), dans lequel l'angle A est compris entre 0° et 90°, de préférence dans une plage de 22° à 75° et la deuxième paroi (302) constitue une forme d'entonnoir conjointement avec le col (307), dans lequel le côté inférieur (307b) du col (307) se trouve à un angle B par rapport à la surface extérieure (302b), dans lequel l'angle B est compris entre 0° et 90°, de préférence dans la plage de 22° à 75°, dans lequel l'angle A est égal à l'angle B.

16. Système d'exhalation (3) selon l'une au moins des revendications précédentes, **caractérisé en ce que** les rainures (304) présentent une section transversale essentiellement rectangulaire, optionnellement avec des coins arrondis et/ou un cercle partiel rapporté, dans lequel les rainures (304) présentent une largeur maximale de 1,25 mm et une profondeur maximale de 1,25 mm.

17. Système d'exhalation (3) selon l'une au moins des revendications précédentes, **caractérisé en ce qu'**au moins trois ergots d'obturation (311) sont disposés sur la surface intérieure (301a) de la première paroi (301) et un nombre correspondant de fentes d'obturation (317) sont disposées dans la surface extérieure (302b) de la deuxième paroi (302), dans lequel les ergots d'obturation (311) de la première paroi (301) sont disposés de façon irrégulière et les fentes d'obturation (317) dans la deuxième paroi (302) sont dans la même mesure disposées de façon irrégulière, de telle façon que la position des fentes d'obturation (317) et celle des ergots d'obturation (311) coïncident uniquement dans une disposition de la première paroi (301) par rapport à la deuxième paroi (302).

18. Interface patient (1) **caractérisé en ce que** l'interface patient (1) comporte un système d'exhalation (3) selon l'une au moins des revendications précédentes et un joint à rotule (6).

19. Interface patient (1) selon la revendication 18, dans lequel le joint à rotule (6) comporte au moins une tête de joint (601) et un palier de joint (602), **caractérisé en ce que** la tête de joint (601) est représentée essentiellement par la troisième paroi (604) et le palier de joint (602) est formé essentiellement par la surface intérieure (302a) de la deuxième paroi (302).

20. Interface patient (1) selon la revendication 19, **caractérisé en ce que** la surface intérieure (302a) de la deuxième paroi (302) est conçue en forme de sphère et la surface extérieure (604b) de la troisième paroi (604) est conçu de façon complémentaire en forme de sphère, dans lequel les rayons de courbure de la surface extérieure (604b) et de la surface intérieure (302a) sont essentiellement identiques et/ou diffèrent légèrement (< 1 %) l'un de l'autre et les rayons de courbure de la surface intérieure (302a) de la deuxième paroi (302) et de la surface extérieure (604b) de la troisième paroi (604) sont sélectionnés de manière à permettre un déplacement de la troisième paroi (604) par rapport à la deuxième paroi (302).
